# NEUE EUROPÄISCHE PATENTSCHRIFT

(11) **EP 3 560 363 B2**
(45) Veröffentlichungstag und Bekanntmachung des Hinweises auf die Entscheidung über den Einspruch: **14.08.2024**
(45) Hinweis auf die Patenterteilung: 24.03.2021
(21) Anmeldenummer: 19169757.2
(22) Anmeldetag: 17.04.2019
(51) Int. Cl.: A24F 47/00, A61M 11/04, A61M 15/06

(54) **VERDAMPFEREINSATZ, VERDAMPFER-TANK-EINHEIT FÜR EINEN INHALATOR, INHALATOR, SOWIE VERFAHREN ZUR FERTIGUNG**
EVAPORATOR INSERT, EVAPORATOR-TANK UNIT FOR AN INHALER, INHALER AND METHOD OF MANUFACTURE
INSERTION D'ÉVAPORATEUR, UNITÉ DE RÉSERVOIR D'ÉVAPORATEUR POUR UN INHALATEUR, INHALATEUR AINSI QUE PROCÉDÉ DE FABRICATION

(30) Priorität: 27.04.2018 DE 102018206647
(43) Veröffentlichungstag der Anmeldung: 30.10.2019
(60) Teilanmeldung: 21156003.2
(73) Patentinhaber: Körber Technologies GmbH, 21033 Hamburg (DE)
(72) Erfinder: ROMMING, Niklas, 22303 Hamburg (DE); JAKLIN, Jan, 79098 Freiburg (DE); ULLNER, Tim, 21039 Hamburg (DE); CORNILS, Lasse, 22111 Hamburg (DE); KALAYDZHYAN, Karen, 22607 Hamburg (DE)
(74) Vertreter: Müller Verweyen

(56) Entgegenhaltungen:
- EP-A1- 3 020 292
- WO-A1-2016/145611
- WO-A1-2017/185051
- WO-A1-2018/024742
- DE-A1- 102016 114 718
- US-B1- 9 795 169

## Beschreibung

Die vorliegende Erfindung betrifft eine Verdampfer-Tank-Einheit für einen Inhalator, einen Inhalator, sowie ein Verfahren zur Fertigung einer Verdampfer-Tank-Einheit und/oder eines Inhalators.

In aktuellen Inhalatoren beziehungsweise elektronischen Zigaretten nach dem Stand der Technik kommen verschiedenste Verdampfereinheiten zum Einsatz, wobei die meisten auf dem Docht-Wendel-Prinzip basieren. Die Verdampfereinheiten weisen verschiedenste Geometrie auf, was unter anderem den unterschiedlichsten Formen und Größen der diversen Inhalatoren am Markt geschuldet ist.

Im Stand der Technik kommt es weiterhin zu teilweisem oder komplettem Trockenlaufen der Verdampfereinheit und zu nicht ausreichender Benetzung der Oberfläche des Verdampfers. In beiden Fällen entstehen durch die fehlende Kühlung durch das zu verdampfende Liquid beziehungsweise die zu verdampfende Flüssigkeit Temperaturen oberhalb von 250 °C, was zur Entstehung von Schadstoffen durch Dekomposition und Radikalisierung der Liquidkomponenten, vor allem Glycerin und Propylenglycol, führt. Durch eine unkontrollierte bzw. nicht erfasste Zufuhr und Verdampfung von Liquid wird die Qualität des entstehenden Aerosols unbeabsichtigt und unkontrolliert beeinflusst.

Zudem sind aktuelle Systeme fertigungsbedingt oft undicht, sodass Flüssigkeit bzw. Liquid auf ungewünschte Weise, zum Beispiel über die Luftzuführung und/oder die Dampfabführung, austreten kann.

In der DE 10 2016 114 718 A1 ist ein Inhalator mit einem Flüssigkeitsspeicher und einem Verdampfer offenbart, wobei der Verdampfer von einer Zugabeeinrichtung umfasst ist, welche in einer wechselbaren Verbrauchseinheit des Inhalators angeordnet ist. Die Verbrauchseinheit ist eine vom Konsumenten auswechselbare Kartusche, in der die Zugabeeinrichtung mit dem Verdampfer herstellerseitig integriert ist.

Die EP 3 020 292 A1 offenbart einen Verdampfer mit einem Grundkörper, einem Mundstück, einer Flüssigkeitskammer, einem Heizelement und einem Einzugselement. Durch das Einzugselement wird eine verdampfbare Flüssigkeit zu einem wärmeabsorbierenden Element geleitet, das wiederum von einem Heizelement erhitzt wird. Die Flüssigkeit wird so durch das wärmeabsorbierende Element verdampft.

Aus der WO 2018/024742 A1 ist ein Einsatz für einen Verdampfer offenbart, der ein sinusförmiges Heizelement aufweist. Darüber wird dann ein wärmeabsorbierendes Material erhitzt, das mit einer Flüssigkeit in Kontakt steht und somit verdampft.

Die Aufgabe der vorliegenden Erfindung ist es, standardisierte Bauteile für einen Inhalator bereitzustellen, die die Anforderungen unterschiedlicher Anbieter erfüllen und sich kostengünstig und durch eine einfache Montage integrieren lassen, sowie entsprechende Fertigungsverfahren anzugeben.

Die Erfindung löst die Aufgabe mit den Merkmalen der unabhängigen Ansprüche.

Erfindungsgemäß weist der Verdampfereinsatz ein Grundteil mit gegenüberliegend angeordneten Stirnseiten und einer um mindestens einen durchströmbaren Strömungskanal angeordneten Mantelseite zwischen den Stirnseiten auf. An der Mantelseite des Grundteils ist mindestens eine Flüssigkeitsöffnung zum Zuführen von verdampfbarer Flüssigkeit von außen in den Verdampfereinsatz zu dem Verdampfer angeordnet. Der Einsatz umfasst somit die für den Verdampfungsvorgang wesentliche Komponenten.

Innerhalb des Verdampfereinsatzes erstreckt sich zwischen den zwei Stirnseiten der Strömungskanal, durch den während des Konsums eines den Verdampfereinsatz umfassenden Inhalators durch einen Konsumenten die Luft beziehungsweise das Aerosol strömt. Der wenigstens eine Verdampfer ist dem Strömungskanal zugeord net und ist dazu eingerichtet, die Flüssigkeit zu erhitzen und dem durch den Strömungskanal strömenden Luftstrom Dampf und/oder Aerosol mit Aroma- und/oder Wirkstoffen zuzugeben.

Die Erfindung erlaubt es, dass das Grundteil von einem Flüssigkeitstank umgebbar ist, aus welchem Flüssigkeit den wenigstens einen Verdampfer zum Verdampfen zuführbar ist. Zwischen den Stirnseiten und vorteilhaft senkrecht zu diesen ist eine Längsachse definiert. Das Grundteil umschließt die Längsachse. Die Längsachse erstreckt sich vorteilhaft zwischen den Stirnseiten und/oder schneidet wenigstens eine der Stirnseiten in einem rechten Winkel. Die Mantelseite bezeichnet die die Längsachse umhüllende, sich in Längsrichtung erstreckende Außenseite des Grundteils.

Das Grundteil ist vorzugsweise patronen- oder hülsenförmig. Die Patronenform beziehungsweise vorteilhaft Hülsenform des Verdampfereinsatzes ist platzsparend und weist vorteilhaft eine neutrale Formgebung auf, was weitestgehende Freiheit bei der Gestaltung eines den Verdampfereinsatz umgebenden externen Teils und/oder eines Inhalators lässt. Aufgrund der Patronen- bzw. Hülsenform des Verdampfereinsatzes ist vorteilhaft eine vielseitige Einbringungsmöglichkeit in jegliche Trankformen ermöglicht. Die Integration der Luft- und/oder Aerosolführung durch den Strömungskanal innerhalb des Verdampfereinsatzes und ein vorteilhaftes Anbindungskonzept durch die Flüssigkeitsöffnung an unterschiedliche Tank-Lösungen liefern eine standardisierte Aerosolqualität und gegebenenfalls eine verbesserte Dichtigkeit der Produkte, beispielsweise der Inhalatoren.

Der Verdampfereinsatz kann in vielen möglichen Produkten zum Einsatz kommen, unabhängig von deren Form, Größe oder dem Tank-Prinzip. Der Flüssigkeitstank kann geschlossen oder offen sein, also ein geschlossenes und/oder offenes Tank-System. Vorzugsweise ist der Verdampfereinsatz als Kapsel ausgeführt. Vorteilhaft trennt das Grundteil den Flüssigkeitstank von dem Verdampfer beziehungsweise Heizer beziehungsweise definiert das Grundteil eine Grenzfläche zwischen im Flüssigkeitstank befindlicher Flüssigkeit und dem Strömungskanal.

Für den Verdampfer kann auf sämtliche bekannte Technologien zurückgegriffen werden und der Verdampfer kann zum Beispiel als Mikro-elektromechanischer bzw. MEMS-Verdampfer, als Docht und/oder als Wendel ausgeführt sein. Der Verdampfer ist in einem Bereich des Strömungskanals angeordnet, der aufgrund seiner Beheizung durch den elektrischen Verdampfer auch als Kamin bezeichnet wird. Stromabwärts vom Kamin, bezogen auf den beim Konsumieren eines mit dem Verdampfereinsatz versehenen Inhalators durch einen Konsumenten typischerweise entstehenden Luftstrom durch den Strömungskanal, wird der Strömungskanal als Schlot bezeichnet.

Erfindungsgemäß ist der Verdampfereinsatz in einem externen Teil, nämlich in einem Flüssigkeitstank, anordenbar und in dieses einschiebbar, um eine einfache und effektive Montage zu erlauben. Der Verdampfereinsatz ist ein Einsatz zum Anordnen und Einschieben, und ggf. zum Eindrehen und/oder Einschrauben, in den Flüssigkeitstank eines Inhalators. Das externe Teil weist vorzugsweise eine Einsetzöffnung, eine Ausgangsöffnung und/oder einen der äußeren Form des Verdampfereinsatzes entsprechend geformten Montagekanal mit einer Einsetzöffnung auf, in welche der Verdampfereinsatz anordenbar und/oder einschiebbar ist.

Bevorzugt weist der Verdampfereinsatz beziehungsweise das Grundteil entlang des Strömungskanals einen abschnittsweise gleichbleibenden monoton verjüngenden Querschnitt auf, um die Montage des Verdampfereinsatzes zu begünstigen. Der Querschnitt muss nicht notwendigerweise rund und kann bspw. mehr- oder vieleckig oder oval sein. Ein wenigstens abschnittsweise unrunder Querschnitt erlaubt eine verdrehsichere Montage des Verdampfereinsatzes.

Bevorzugt umfasst der Verdampfereinsatz mindestens ein Halte- und/oder Befestigungselement zur Halterung und/oder Befestigung des Verdampfereinsatzes in dem externen Teil, um ein unbeabsichtigtes Lösen des Verdampfereinsatzes in dem externen Teil zu verhindern. Der Verdampfereinsatz kann auch ein Gegenstück zu einem Halte- und/oder Befestigungselement in dem externen Teil vorsehen. Die Befestigung des Verdampfereinsatzes in dem externen Teil kann lösbar oder unlösbar sein.

Bevorzugt ist der Verdampfereinsatz in das externe Teil verdrehsicher einbaubar, um zu gewährleisten, dass die Flüssigkeitsöffnung einer entsprechenden Zuführöffnung im externen Teil zugeordnet ist und/oder die elektrischen Kontakte des Verdampfereinsatzes in eine Wirkverbindung mit elektrischen Kontakten eines Komponententeils des Inhalators in Verbindung gebracht werden können, um beispielsweise eine Versorgung mit elektrischer Energie aus einem Energiespeicher herzustellen. Vorzugsweise umfasst der Verdampfereinsatz ein Verdrehsicherungselement an der Mantelseite des Grundteils, wie beispielsweise Rastelement, Nase, Nut, und/oder Aussparung.

In einer bevorzugten Ausführungsform sind die elektrischen Kontakte an einer der Stirnseiten des Verdampfereinsatzes angeordnet, um eine einfache elektrische Verbindung mit einem externen Bauteil, beispielsweise einem Komponententeil mit einem Energiespeicher herstellen zu können.

Es ist von Vorteil, dass in dem Grundteil mindestens eine luftdurchlässige Be- und/oder Entlüftungseinrichtung zur Be- und/oder Entlüftung eines Flüssigkeitstanks vorgesehen ist, um ungewollte Druckunterschiede zwischen dem Flüssigkeitstank und dem Strömungskanal zu vermeiden. Durch die Verdampfung von aus dem Flüssigkeitstank zugeführter Flüssigkeit entsteht in dem Flüssigkeitstank ein Unterdruck bzw. am Verdampfer ein Druckgefälle, das dazu führen kann, dass Liquid vom Verdampfer weggedrückt wird und dieser damit trocken fällt. Ein Druckgefälle am Verdampfer kann außerdem dadurch entstehen, dass das Gerät auf große Höhen befördert wird, zum Beispiel beim Transport in einem Flugzeug, wobei der verringerter Außendruck zu einem unkontrollierten Austritt von Liquid aus dem Flüssigkeitstank führen kann. Ein ungewollter Unterdruck kann auch durch das Saugen des Konsumenten an einem Mundende des Inhalators entstehen, während der Verdampfer nicht beheizt ist. Dies führt zu Liquidaustritt durch den Verdampfer. Dies kann durch eine Be- und/oder Entlüftung des Flüssigkeitstanks über den Schlot des Verdampfereinsatzes verhindert werden, da dann gleichzeitig am Flüssigkeitstank und damit an der Rückseite beziehungsweise der dem Flüssigkeitstank zugewandten des Verdampfers ein Unterdruck anliegt. Vorzugsweise ist die Be- und/ oder Entlüftung so ausgelegt, dass unabhängig von der räumlichen Orientierung des Verdampfereinsatzes gewährleistet ist, dass nur Luft durch die Be- und/oder Entlüftungseinrichtung treten kann und der Durchtritt von Liquid unterbunden ist.

Vorzugsweise umfasst der Strömungskanal Vor- und/oder Nachbehandlungsbereiche zur Vor- und/oder Nachbehandlung durch den Strömungskanal strömender Luft und/oder verdampfter Flüssigkeit, um eine verbesserte Aerosol-Dampf-Gemisch-Qualität zu erzielen. Eine Vorbehandlung findet vorzugsweise stromaufwärts des Kamins statt. Eine Nachbehandlung findet vorzugsweise stromabwärts des Kamins im Schlot statt. Das am Verdampfer entstehende Aerosol-Dampf-Gemisch kann auf dem Weg in Richtung eines sich stromabwärts vom Verdampfer befindenden Mundstückes im Kamin und/ oder im Schlot durch Vor- und/oder Nachbehandlungsvorrichtungen nachbehandelt werden. So sind beispielsweise Luftzuführungen denkbar, um eine Durchmischung mit Nebenluft zu gewährleisten und/oder durch laminare Strömungen einen Kontakt mit der Schlotwand bzw. Strömungskanalwand und damit eine eventuelle Kondensation zu vermeiden. Es ist auch denkbar, dass die Vor- und/oder Nachbehandlungsvorrichtungen dazu eingerichtet sind, die Aufströmung des Luftstroms auf den Verdampfer zu beeinflussen. Dazu können die Vor- und/oder Nachbehandlungsbereiche beispielsweise Luftleitelemente aufweisen. Die Luftzuführung ist dazu eingerichtet, dem Luftstrom im Strömungskanal vorzugsweise kühlere Frischluft zuzugeben. Auch vorgewärmte Luft ist denkbar, d.h. der Luftstrom wird vor dem Auftreffen auf den Verdampfer erwärmt. Eine mögliche Nachbehandlung umfasst eine Nacherwärmung des Luftstroms nach dem Passieren des Verdampfers.

In einer bevorzugten Ausführungsform umfasst der Verdampfereinsatz eine Mehrzahl von elektrischen Verdampfern, um die Verdampferleistung den Anforderungen und Eigenschaften des jeweiligen Liquides anpassen zu können.

Vorzugsweise sind die elektrischen Verdampfer entlang des Umfangs der Mantelseite des Grundteils und/oder entlang des Strömungskanals angeordnet, um eine gleichmäßige und effektive Verdampfung innerhalb des Strömungskanals zu ermöglichen.

Bevorzugt weist der Verdampfereinsatz eine Mehrzahl von Flüssigkeitsöffnung auf, wobei jedem elektrischen Verdampfer Flüssigkeit aus einer entsprechenden Flüssigkeitsöffnung zuführbar ist, um den Transport von Flüssigkeit zu jedem Verdampfer zu gewährleisten. Bevorzugt können verschiedenen Verdampfern Flüssigkeiten aus verschiedenen Flüssigkeitstanks beziehungsweise Kammern eines Flüssigkeitstanks zugeführt werden, um eine Mischung und/oder Auswahl von Aroma- und/oder Wirkstoffen zu ermöglichen.

Bevorzugt weist der Verdampfereinsatz an der Mantelseite des Grundteils mindestens ein Halteelement, beispielsweise einen Dorn zur Halterung eines kapillaren Elementes auf, um die Flüssigkeitsöffnung unabhängig von der Orientierung des Verdampfereinsatzes mit Flüssigkeit versorgen zu können. Das kapillare Element, beispielsweise ein Schwamm, ist dazu eingerichtet, Flüssigkeit zu speichern und dem Liquidzuführelement zuzuführen. Damit kann auch bei kurzzeitigem Betrieb eines Inhalators mit dem Verdampfereinsatz über Kopf ausreichend Liquid bereitgestellt werden, um den Konsum des Inhalators zu ermöglichen.

Vorzugsweise umfasst der Verdampfereinsatz wenigstens einen vom Strömungskanal separaten Nebenluftkanal, um Frischluft führen zu können und/oder Druckmessungen unabhängig von der Verdampfung beziehungsweise Aerosolbildung gewährleisten zu können. Vorzugsweise umfasst der Verdampfereinsatz eine Separationswand und die Separationswand ist dazu eingerichtet, den Strömungskanal vom Nebenluftkanal zu trennen. Der Nebenluftkanal ist vorteilhaft ein Bypass zum Strömungskanal, der auch als Hauptluftkanal bezeichnet werden kann. Vorzugsweise ist der Querschnitt des Nebenluftkanals wenigstens abschnittweise kleiner als der Querschnitt des Hauptluftkanals.

In einer bevorzugten Ausführungsform kommuniziert die Flüssigkeitsöffnung mit einem Liquidzuführelement, wobei das Liquidzuführelement dazu eingerichtet ist, Flüssigkeit zum Verdampfer zu transportieren, eine leckagefreie und kontrollierte Zufuhr von Flüssigkeit bzw. Liquid zu ermöglichen. Das Liquidzuführelement, beispielsweise ein Docht, ist vorzugsweise ein kapillares Element, welches dazu eingerichtet ist, die Flüssigkeit mittels Kapillarkräften dem Verdampfer zuzuführen. Beispielsweise kann das Liquidzuführelement ein poröses Medium, Docht oder Ähnliches sein. Das Liquidzuführelement kann vorzugsweise in bzw. von dem Grundteil gehalten sein. Der Verdampfereinsatz ist somit vorzugsweise eine Kapsel und umfasst vorzugsweise sowohl den Heizer als auch den Docht beziehungsweise ein Teil des Dochtsystems.

Bevorzugt umfasst der Verdampfereinsatz eine elektronische Einheit zum Steuern und/oder Regeln des Verdampfers und/oder zur Speicherung von Kenndaten, um beispielsweise die Steuerung und/oder Regelung mit der Identifizierung des Verdampfereinsatzes verknüpfen zu können. Jegliche Art der Identifizierung ist möglich, beispielsweise passive elektronische Methoden, etwa Widerstandsnetzwerke.

Eine erfindungsgemäße Verdampfer-Tank-Einheit umfasst einen Flüssigkeitstank und einen in dem Flüssigkeitstank eingesetzten Verdampfereinsatz. Der Flüssigkeitstank weist eine Einsetzöffnung und eine Ausgangsöffnung auf, wobei sich der Verdampfereinsatz von der Einsetzöffnung bis zur Ausgangsöffnung erstreckt. Dies erlaubt das funktionsgemäße Montieren beziehungsweise Einschieben des Verdampfereinsatzes in die Einsetzöffnung und die Ausgangsöffnung. Der Verdampfereinsatz kann beispielsweise aus der Ausgangsöffnung aus dem Flüssigkeitstank austreten, mit dem Flüssigkeitstank fluchten oder innerhalb des Flüssigkeitstanks mit der Ausgangsöffnung abschließen.

Vorzugsweise ist der Verdampfereinsatz so in den Flüssigkeitstank einführbar, dass der Flüssigkeitstank die Mantelseite des Verdampfereinsatzes wenigstens in dem Abschnitt der Flüssigkeitsöffnung umschließt. Dadurch ist sichergestellt, dass die Flüssigkeit aus dem Flüssigkeitstank durch in dem Flüssigkeitstank vorgesehene Zuführöffnungen und die Flüssigkeitsöffnungen des Verdampfereinsatzes zum Verdampfer transportiert werden kann.

In einer bevorzugten Ausführungsform ist der Flüssigkeitsöffnung ein kapillares Element innerhalb des Flüssigkeitstanks zugeordnet, um beispielsweise orientierungsunabhängig Flüssigkeit zu speichern, einen orientierungsunabhängigen Flüssigkeitstransport aus dem Flüssigkeitstank zu der Flüssigkeitsöffnung zu gewährleisten und/oder ein unkontrolliertes Austreten von Liquid zu vermeiden.

Der Verdampfereinsatz ist in der Verdampfer-Tank-Einheit verdrehsicher gehalten, um beispielsweise eine eindeutige Zuordnung der im Flüssigkeitstank vorgesehenen Zuführöffnung und den Flüssigkeitsöffnungen und/oder den Kontakten und entsprechenden Kontakten eines weiteren, externen Komponententeils beziehungsweise Bauteil des Inhalators zu ermöglichen. Der Verdampfer weist in dieser Ausführungsform vorzugsweise ein Verdrehsicherungselement auf. Der Verdampfereinsatz kann auch ein Gegenstück zu einem Verdrehsicherungselement in dem externen Teil vorsehen. Die Verdrehsicherung kann auch durch einen nicht-rotationssymmetrischen beziehungsweise unrunden Querschnitt des Grundteils gebildet sein.

Bevorzugt umfasst der Flüssigkeitstank eine Mehrzahl voneinander separater Kammern, wobei jedem elektrischen Verdampfer Flüssigkeit aus einer entsprechenden Kammer zuführbar ist, um den Transport von vorzugsweise verschiedenen in den Kammern gespeicherten Flüssigkeiten zu dem Verdampfer zu ermöglichen.

Optional kann der Flüssigkeitstank mindestens ein luftdurchlässiges Be- und/oder Entlüftungselement aufweisen, um die Bildung eines Unterdrucks im Flüssigkeitstank zu verhindern und einen Druckausgleich sowie eine zuverlässige Förderung von Flüssigkeit zu gewährleisten.

Vorteilhaft fluchtet wenigstens eine der Stirnseiten des Verdampfereinsatzes mit einer entsprechenden Stirnseite des Flüssigkeitstanks, um eine geometrisch abgeschlossene Verdampfer-Tank-Einheit bereitstellen zu können.

Der Verdampfereinsatz wird in die Einsetzöffnung und bis zu der Ausgangsöffnung des Flüssigkeitstanks eingesetzt, insbesondere eingeschoben, um eine einfache Montage des Verdampfereinsatzes und dem Flüssigkeitstank zu ermöglichen.

Vorzugsweise wird der Verdampfereinsatz mit der auslassseitigen Stirnseite bis zur Ausgangsöffnung geführt, insbesondere geschoben, anschließend wird der Flüssigkeitstank über eine sich zwischen dem Grundteil und der Einsetzöffnung angeordnete oder verbleibende Befüllöffnung mit Flüssigkeit befüllt, und anschließend wird der Verdampfereinsatz unter Schließung der Befüllöffnung bis zu einem Anschlag durch die Einsetzöffnung geführt, insbesondere geschoben. Der Anschlag kann beispielsweise durch einen Kragen an der einlassseitigen Stirnseite des Verdampfereinsatzes, Formgebung des Grundteils, der Einsetzöffnung und/oder der Ausgangsöffnung ausgebildet sein. In einer bevorzugten Ausführungsform kann der Anschlag durch die Bodenplatte des Verdampfereinsatzes ausgebildet sein, beispielsweise wenn diese kragenförmig über das Grundteil übersteht. Auch eine konische Ausführung des Kragens ist möglich. Der Einsetzöffnung und/oder der Ausgangsöffnung können vorteilhaft Dichtelemente zugeordnet sein, um eine leckagefreie Verdampfer-Tank-Einheit bereitstellen zu können.

Die Erfindung wird im Folgenden anhand bevorzugter Ausführungsformen unter Bezugnahme auf die beigefügten Figuren erläutert. Dabei zeigt
- Fig. 1: einen Längsschnitt durch einen Verdampfereinsatz;
- Fig. 2: einen Längsschnitt durch eine Verdampfer-Tank-Einheit;
- Fig. 3: einen Längsschnitt durch eine Verdampfer-Tank-Einheit mit einem Verdampfereinsatz mit einem Dorn;
- Fign. 4, 5: einen Längs- und einen Querschnitt durch eine Verdampfer-Tank-Einheit mit einem Zweikammer-Flüssigkeitstank und einem Verdampfereinsatz mit zwei Verdampfern;
- Fign. 6, 7: eine perspektivische Ansicht und einen Längsschnitt einer Verdampfer-Tank-Einheit mit einem eckigen Querschnitt;
- Fig. 8A, 8B: eine perspektivische Ansicht und einen Längsschnitt einer Verdampfer-Tank-Einheit mit einem runden Querschnitt;
- Fign. 9 - 11: perspektivische Ansichten einer Verdampfer-Tank-Einheit mit einem Mundende;
- Fig. 12: eine schematische Darstellung eines Inhalators;
- Fig. 13: eine schematische Darstellung eines Verdampfers mit einem Liquidzufuhrelement und einem Flüssigkeitstank;
- Fig. 14: einen Längsschnitt durch einen Verdampfereinsatz mit einem Nebenluftkanal; und
- Fig. 15: einen Längsschnitt durch eine Verdampfer-Tank-Einheit in einer vorteilhaften Ausführungsform.

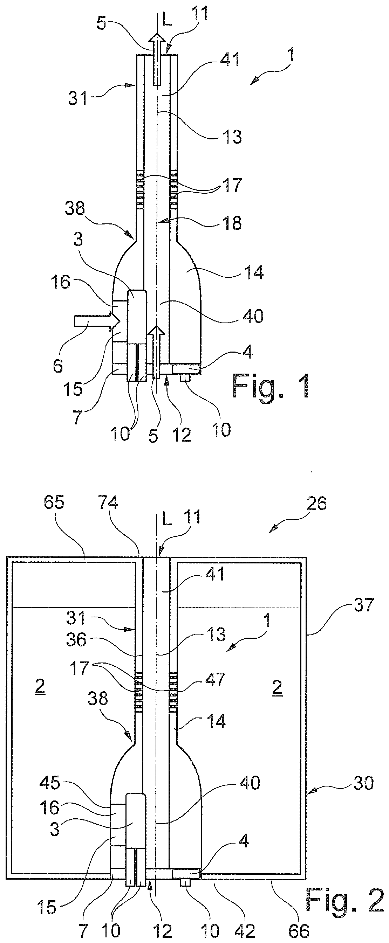

Figur 1 zeigt einen Verdampfereinsatz 1 für einen Inhalator 27 umfassend einen elektrischen Verdampfer 3 zum Verdampfen von dem Verdampfer 3 zugeführter Flüssigkeit 2. Der Verdampfereinsatz 1 weist gegenüberliegend angeordnete Stirnseiten 11, 12 und ein um einen durchströmbaren Strömungskanal 13 angeordnetes Grundteil 14 zwischen den Stirnseiten 11, 12 auf. Entlang des Strömungskanals 13 und/oder zwischen den Stirnseiten 11, 12 ist eine Längsachse L definiert. Das Grundteil 14 kann sich vorzugsweise entlang der Längsachse L bzw. des Strömungskanals 13 zwischen den Stirnseiten 11, 12 erstrecken.

Der Verdampfereinsatz 1 ist patronen- oder hülsenförmig. In diesem Beispiel weist das Grundteil 14 einen abschnittsweise runden Querschnitt auf und die Längserstreckung entlang des Strömungskanals 13 ist größer als der Durchmesser des Grundteils 14. Damit ist der Verdampfereinsatz 1 länglich. Vorzugsweise hat der Verdampfereinsatz 1 eine Länge von 0,1 cm bis 10 cm, weiter vorzugsweise 0,5 cm bis 5 cm, beispielsweise von 2 cm und einen Durchmesser von vorzugsweise 0,1 cm bis 1 cm, weiter vorzugsweise 0,25 cm bis 0,75 cm, beispielsweise 0,5 cm. Entlang der Längsachse L des Verdampfereinsatzes 1 weist das Grundteil 14 einen hohlzylinderförmigen Abschnitt 41 und einen sich nicht notwendigerweise verjüngenden Abschnitt 40 mit einem stromabwärts abnehmenden Durchmesser beziehungsweise Querschnitt auf.

Beim Inhalieren bzw. Konsumieren zieht ein Konsument an einem Mundende 39 des Inhalators 27, wobei ein Saugdruck entsteht, der einen Luftstrom 5 durch den Strömungskanal 13 bedingt. Die Luft tritt an einer dem Mundende 39 abgewandten Stirnseite 12 in den Strömungskanal 13, durchströmt den Strömungskanal 13 und tritt an einer dem Mundende 39 zugewandten Stirnseite 11 aus dem Strömungskanal 13 aus. In dem Strömungskanal 13 wird dem Luftstrom 5 verdampfte Flüssigkeit 2 zugegeben, um dem Konsumenten ein Aerosol bzw. Aerosol-Dampf-Gemisch zu verabreichen.

Der Luftkanal 13 umfasst einen als Kamin 40 bezeichneten Abschnitt, der dem Verdampfer 3 zugeordnet ist, und einen vom Verdampfer 3 stromabwärts angeordneten Schlot 41. Der Schlot 41 kann vom Anwender und/oder Hersteller je nach erforderlicher Länge entsprechend gestutzt werden bzw. kürzbar sein.

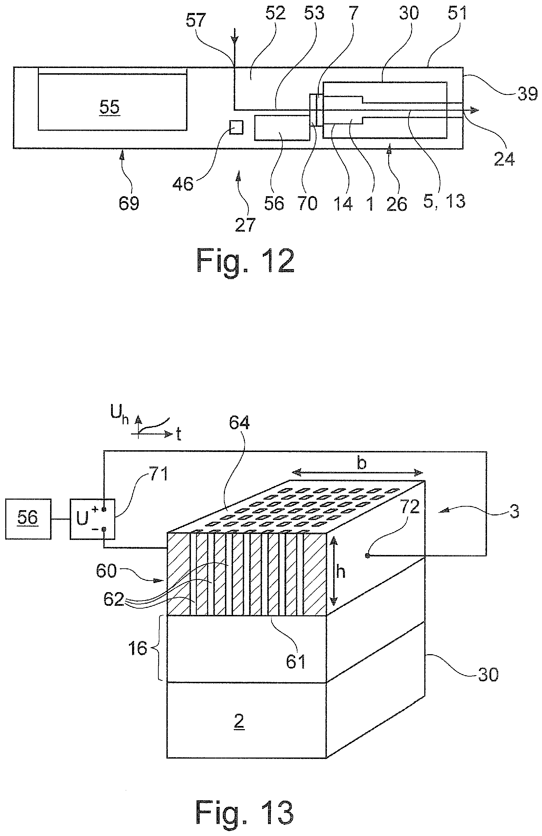

In dem Grundteil 14 ist mindestens eine Flüssigkeitsöffnung 15 zum Zuführen von verdampfbarer Flüssigkeit 2 radial von außen in den Verdampfereinsatz 1 zu dem Verdampfer 3 angeordnet. Der Flüssigkeitsöffnung 15 ist ein Liquidzuführelement 16 zugeordnet. Das Liquidzuführelement 16 dient der Zuführung der Flüssigkeit 2 durch die Flüssigkeitsöffnung 15 hin zum Verdampfer 3, siehe beispielsweise Figur 13 und der dazugehörigen Beschreibung.

Der Verdampfereinsatz 1 umfasst elektrische Kontakte 10 zur elektrischen Kontaktierung des Verdampfereinsatzes 1 für die externe Versorgung mit elektrischer Energie und/oder zum Empfangen von Steuersignalen für den Verdampfer 3. In diesem Ausführungsbeispiel sind die Kontakte 10 an einer stromaufwärts des Verdampfers 3 angeordneten Bodenplatte 7 des Verdampfereinsatzes 1 angeordnet. Die Kontakte 10 können beispielsweise zur Beheizung des Verdampfers 3 eine elektrische Verbindung zwischen dem Verdampfer 3 und einem Komponententeil des Inhalators 27 herstellen, welches einen Energiespeicher 55 umfasst. Die Kontakte 10 können beispielsweise eine elektrische Verbindung zwischen einer elektronischen Einheit 4 des Verdampfereinsatzes 1 und einer im Inhalator 27 vorgesehenen elektronischen Steuerungsvorrichtung 56 herstellen, um Daten, beispielsweise Kennungen, Füllständen, Betriebszeiten usw. austauschen zu können.

Die vorzugsweise standardisierte, elektrische Kontaktierung zwischen dem Verdampfereinsatz 1 und/oder einer Verdampfer-Tank-Einheit 26 bzw. einer Kartusche und/oder einem Komponententeil des Inhalators 27 dient beispielsweise der Ansteuerung des oder der Verdampfer 3 und/oder einer Identifizierung der Kartusche über einen beispielsweise in der elektronischen Einheit 4 vorgesehenen ID-Chip oder eine andere Identifizierungsmethode. Die elektronische Einheit 4 kann über eine serielle Schnittstelle, beispielsweise über einen Eindraht-Bus, an das Komponententeil angeschlossen werden und/oder gemeinsame Kontakte 10 mit den Verdampfer 3 verwenden. Vorzugsweise sind die Kontakte 10 in einer verdrehsicheren Standard-Anordnung, zum Beispiel in Form von konzentrischen Kontaktpads für Federstifte definiert. Es ist aber auch eine kontaktlose Identifizierungsmethode, beispielsweise über RFID und/oder Nahfeldkommunikation (NFC) denkbar.

Der Verdampfereinsatz 1 ist vorzugsweise aus einem Kunststoff gefertigt und/oder die Bodenplatte 7 umfasst eine Leiterplatine (PCB). Die Bodenplatte 7 kann demontierbar oder fest mit dem Grundteil 14 verbunden sein. Die Bodenplatte 7, der Kamin 40 und der Schlot 41 definieren vorzugsweise eine standardisierte Baueinheit, die für verschieden geformte Flüssigkeitstanks 30 geeignet ist. Es sind auch Ausführungsformen ohne Bodenplatte 7 denkbar.

In dem Strömungskanal 13 kann mindestens ein Vor- und/oder Nachbehandlungsbereich 18 mit einer nicht gezeigten Vor- und/oder Nachbehandlungseinrichtung vorgesehen sein. In dem Vor- und/ oder Nachbehandlungsbereich 18 kann beispielsweise ein obstruktives Element oder Schwamm, der ein direktes Austreten von großen Tröpfchen oder Liquid verhindert, standardmäßig vorgesehen sein. Ein Luftführungs- und/oder Widerstandselement kann in ausgesuchten und geeigneten Bereichen des gesamten Strömungskanals 13 vorgesehen sein. Ferner kann als Vor- und/oder Nachbehandlungseinrichtung ein Heiz- und oder Kühlelement zur Nachbehandlung des Aerosols eingesetzt werden.

Der Luftstrom 5 kann durch entsprechend ausgeformte Aushöhlungen auf der Innenseite des Grundteils 14 beziehungsweise im Luftkanal 13 beeinflusst und die Aufströmung der Luft auf den Verdampfer 3 definiert werden. Die Aushöhlungen können den Vor- und/oder Nachbehandlungsbereichen 18 zugeordnet sein. So kann beispielsweise eine laminare Querströmung der Luft über die Oberfläche des Verdampfers 3, eine senkrechte Aufströmung und/oder eine tangentiale Quer-Einströmung erzielt werden. Der Öffnungsquerschnitt der Luftzufuhr kann den Luftstrom 5 beim Auftreffen auf den Verdampfer 3 und die Aerosolqualität, wie beispielsweise die Durchmischung und/oder Tröpfchengröße beeinflussen.

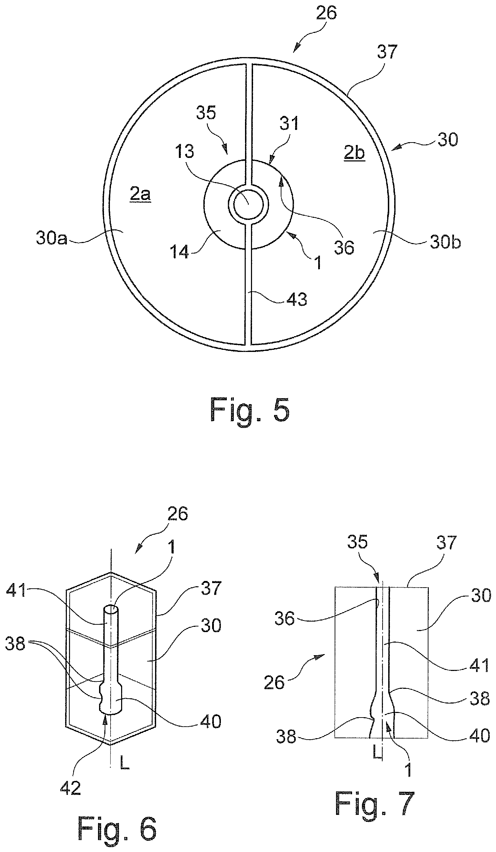

Figur 2 zeigt eine erfindungsgemäße Verdampfer-Tank-Einheit 26 umfassend einen Verdampfereinsatz 1 und einen Flüssigkeitstank 30. Der Flüssigkeitstank 30 weist ein Gehäuse 37 und beispielsweise einen in Figuren 7 bis 11 angedeuteten Montagekanal 35 auf und umschließt diesen in Umfangsrichtung. Der optionale Montagekanal 35 kann in Wirkverbindung mit einer Einsetzöffnung 42 und einer Ausgangsöffnung 74 stehen, in die ein Verdampfereinsatz 1 anordenbar, insbesondere einschiebbar beziehungsweise einsetzbar ist. Der Montagekanal 35 wird vorzugsweise von einer Innenfläche 36 des Flüssigkeitstanks 30 beziehungsweise des Gehäuses 37 definiert. Das Grundteil 14 weist eine Mantelseite 31 auf, wobei die Innenfläche 36 an die Mantelseite 31 angepasst ist. Vorzugsweise kontaktiert die Mantelseite 31 des Grundteils 14 die Innenfläche des Montagekanals formschlüssig, um eine platzsparende und leckagefreie Montage zu ermöglichen. Vorteilhaft ist der Verdampfereinsatz 1 entlang der Längsachse L in den Montagekanal 35 einsetzbar beziehungsweise einschiebbar.

In anderen Ausführungsformen wird der Montagekanal 35 durch Einpassen des Verdampfereinsatzes 1 in den Flüssigkeitstank 30 definiert, wobei die Mantelseite 31 den Flüssigkeitstank 30 in der Verdampfer-Tank-Einheit 1 schließt und Leckagefreiheit gewährleistet.

Die Innenfläche 36 des Montagekanals 35 weist eine mit der Flüssigkeitsöffnung 15 eines in den Montagekanal 35 eingeschobenen Verdampfereinsatzes 1 korrespondierende Zuführöffnung 45 auf. Die Zuführöffnung 45 wird bei der Montage der Verdampfer-Tank-Einheit 26 mit der Flüssigkeitsöffnung 15 in Deckung gebracht, damit Flüssigkeit 2 aus dem Flüssigkeitstank 30 dem Verdampfer 3 zugeführt werden kann.

In dem Strömungskanal 13, beispielsweise im Schlot 41 kann mindestens eine Be- und/oder Entlüftungseinrichtung 17 vorgesehen sein. Die Be- und/oder Entlüftungseinrichtung 17 kann beispielsweise entlang des Schlots 41 eine Mehrzahl von Perforationen aufweisen. Um ein Austritt von Liquid durch die Löcher beziehungsweise Perforationen zu vermeiden, kann der Schlot 41 bzw. das Grundteil 14 durch eine geeignete Materialwahl, die Ausnutzung des Lotuseffekts oder eine Strukturierung der Oberfläche, beispielsweise einer Nanostrukturierung, so beschaffen sein, dass ein möglichst großer Kontaktwinkel zwischen der Oberfläche und dem Liquid besteht und die Oberfläche flüssigkeitsabweisend wird. Zusätzlich kann in den Schlot 41 eintretendes Liquid über ein kapillares Element 33 aufgefangen werden und damit eine Leckage vermieden werden.

Vorzugsweise weist der Flüssigkeitstank 30 mindestens ein luftdurchlässiges Be- und/oder Entlüftungselement 47 auf. Das Be- und/oder Entlüftungselement 47 ist in der montierten Verdampfer-Tank-Einheit 26 vorzugsweise in Deckung mit den Be- und/oder Entlüftungseinrichtungen 17, um funktionell zusammenzuwirken. Das Grundteil 14 kann auch aus einem semipermeablen Material bestehen, was keine Flüssigkeit 2 in den Strömungskanal 13 eintreten lässt und gleichzeitig eine Be- und/oder Entlüftung des Flüssigkeitstanks 30 erlaubt.

Der Verdampfereinsatz 1 weist vorzugsweise ein Halte- und/oder Befestigungselement 38 zur Befestigung und Halterung des Verdampfereinsatzes 1 in der Einsetzöffnung 42, der Ausgangsöffnung 74 und/oder dem Montagekanal 35 beziehungsweise in dem Flüssigkeitstank 30 auf. Das Halte- und/oder Befestigungselement 38 kann beispielsweise eine Aussparung, Ausbeulung, Nase, Nut und/ oder ein Rastelement aufweisen. Das Halte- und/oder Befestigungselement 38 kann einseitig und/oder nicht-rotationssymmetrisch sein, um eine verdrehsichere Montage des Verdampfereinsatzes 1 im Flüssigkeitstank 30 zu ermöglichen. Das Halte- und/oder Befestigungselement 38 bewirkt die ortsfeste Halterung des Verdampfereinsatzes 1 im Flüssigkeitstank 30 und verhindert ein Verschieben und/oder Verdrehen des Verdampfereinsatzes 1. Das Halte- und/oder Befestigungselement 38 kann, wie in dem Ausführungsbeispiel gezeigt durch die Formung der Mantelseite 31 des Mantelgehäuses 14 gebildet sein. Das gezeigte Halte- und/oder Befestigungselement 38 umfasst eine Verjüngung des Grundteils 14 und verhindert ein Verschieben in Richtung der Längsachse L des Verdampfereinsatzes 1.

Das Halte- und/oder Befestigungselement 38 kann den Verdampfereinsatz 1 unlösbar in dem Flüssigkeitstank 30 halten, bspw. durch Verschweißen, so dass die Verdampfer-Tank-Einheit 26 ein Produkt ist, in dem der Verdampfereinsatz 1 fest und für den Konsumenten unlösbar verbaut ist. Alternativ ist der Einsatz in Offen-TankSystemen, die einen durch den Nutzer wiederbefüllbaren Flüssigkeitsspeicher 30 aufweisen, denkbar.

Das Halte- und/oder Befestigungselement 38 kann so gestaltet sein, dass der Verdampfereinsatz 1 in die Einsetzöffnung 42, die Ausgangsöffnung 74 und/oder den Montagekanal 35 bzw. in den Flüssigkeitstank 30 einsetzbar und herausnehmbar ist. Dies erlaubt eine reversible Montage der Verdampfer-Tank-Einheit 26 und ein Auswechseln des Verdampfereinsatzes 1 unter Beibehaltung des Flüssigkeitstanks 30, womit der Flüssigkeitstank 30 und/oder der Verdampfereinsatz 1 als Mehrwegteil ausgeführt sein kann. Mit den erläuterten Ausführungsformen ist die Integration des Verdampfereinsatzes 1 in verschiedene Flüssigkeitstanks 30 mit unterschiedlichen Tanksystemen denkbar.

Die Grundform des patronenförmigen Verdampfereinsatzes 1 ist dazu eingerichtet, dass der Verdampfereinsatz 1 in verschiedene Flüssigkeitstanks 30 mit nur minimalen Anpassungen und vorzugsweise wenigen mechanischen Schnittstellen eingebaut werden kann. Vorteilhaft ist der Verdampfereinsatz 1 mit dem Flüssigkeitstank 30 dichtend verbunden, um eine Flüssigkeitsleckage an der Verbindungsstelle zu verhindern. Die Verbindung zwischen dem Verdampfereinsatz 1 und dem Flüssigkeitstank 30 kann beispielsweise durch Presspassung, Schweißen und/oder Kleben realisiert werden.

Durch die Integration der Verdampferfunktion und der elektronischen Steuerung in den Verdampfereinsatz 1 sind die funktionellen Anforderungen an den Flüssigkeitstank 30 gering und beschränken sich auf die Aufnahme des Liquids bzw. der Flüssigkeit 2. Dies lässt die Formgebung des Flüssigkeitstanks 30 bzw. des Gehäuses 37 offen, da vorzugsweise die einzige Beschränkung der Gehäuseform 37 die Aussparung für die Einsetzöffnung 42, die Ausgangsöffnung 74 und/oder des Montagekanals 35 ist.

Es ist auch eine Ausführungsform denkbar, bei der eine Mehrzahl von Verdampfereinsätzen 1, bspw. in Längsrichtung seriell, beispielsweise in den Montagekanal 35, und/oder in eine Mehrzahl von Montagekanälen, einsetzbar sind. D.h. die Verdampfereinsätze 1 sind in den Ausführungsformen in Reihe seriell oder parallel/nebeneinander einsetzbar. Beispielsweise wird ein gemeinsamer Luftstrom 5 durch mehrere Verdampfereinsätze 1 seriell geführt. Mehrere Verdampfereinsätze 1 können auch in mehrere Flüssigkeitstanks 30 und/oder mehrere Kammern eines Flüssigkeitstanks 30 einsetzbar sein, um eine Kombination von Aroma- und/oder Wirkstoffen zu erzielen.

Die einlassseitige Stirnseite 12 des Verdampfereinsatzes 1, gegebenenfalls die Bodenplatte 7, fluchtet vorteilhaft mit einer einlassseitigen Stirnseite 65 des Gehäuse 37. Die auslassseitige Stirnseite 11 des Verdampfereinsatzes 1 fluchtet vorteilhaft mit einer auslassseitigen Stirnseite 66 des Gehäuse 37. Hierdurch wird eine kompakte Bauform der Verdampfer-Tank-Einheit 26 erzielt.

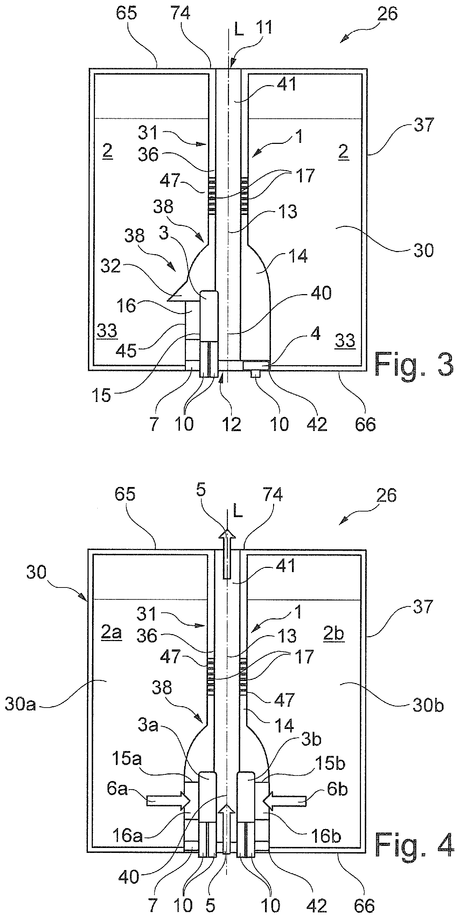

Figur 3 zeigt eine Verdampfer-Tank-Einheit 26 mit einem Verdampfereinsatz 1 mit einem an der Mantelseite 31 des Grundteils 14 vorgesehenen Dorn 32, der zur Halterung eines kapillares Elementes 33 innerhalb eines Flüssigkeitstanks 30 vorgesehen sein kann.

Der Dorn 32 kann als Halte- und/oder Befestigungselement 38 dienen und eine verschiebefeste und/oder verdrehsichere Montage des Verdampfereinsatzes 1 im Flüssigkeitstank 30 gewährleisten. Der Dorn 32 kann auch vollumfänglich als Kragen an der Mantelseite 31 des Grundteils 14 gebildet sein. Der Dorn 32 beziehungsweise die Lippe ist vorteilhaft schmaler als der Abschnitt des Grundteils mit dem größten Umfang beziehungsweise als der der Bodenplatte 7 zugeordnete Kragen, um eine Montage des Verdampfereinsatzes 1 durch Einschub in den Flüssigkeitstank 30 zu ermöglichen.

Das kapillare Element 33 ist dazu eingerichtet, Flüssigkeit 2 zu speichern und dem Liquidzuführelement 16 zuzuführen. Die Verwendung eines kapillaren Elements 33 bzw. eines porösen Mediums erlaubt Speicherung und/oder Pufferspeicherung von Flüssigkeit 2 an einem durch das kapillare Element 33 definierten Ort unabhängig von der Orientierung und/ oder dem Füllstand des Flüssigkeitstanks 30 bzw. der Kartusche. Das kapillare Element 33 erstreckt sich wenigstens abschnittsweise und/oder vollständig innerhalb des Flüssigkeitstanks 30.

Figuren 4 und 5 zeigen eine Verdampfer-Tank-Einheit 26 mit mehreren, hier beispielsweise zwei Kammern 30a, 30b in einem Flüssigkeitstank 30 und einem Verdampfereinsatz 1 mit hier zwei Verdampfern 3a, 3b, zwei Flüssigkeitsöffnungen 15a, 15b, zwei Liquidzuführelementen 16a, 16b und zwei Flüssigkeitsströmen 6a, 6b, welche den Verdampfern 3a, 3b zwei vorzugsweise verschiedene Flüssigkeiten 2a, 2b aus den Kammern 30a, 30b zum Verdampfen zuführen. Der Flüssigkeitstank 30 ist in diesem Beispiel durch eine Trennwand 43 segmentiert und dazu eingerichtet, verschiedene Flüssigkeiten 2a, 2b zu speichern. Bei der Verwendung von mehreren Kammern 30a, 30b kann eine beliebige Tankgeometrie verwendet werden, bei der Trennwände 43 zur Trennung der Flüssigkeiten 2a, 2b vorgesehen sind.

Vorzugsweise sind mehrere Liquidzuführelemente 16a, 16b in Umfangsrichtung des Grundteils 14 versetzt am Grundteil 14 angeordnet und kommunizieren mit der Flüssigkeitsöffnung 15 oder mehreren Flüssigkeitsöffnungen 15a, 15b. Die Liquidzuführelemente 16a, 16b können in Umfangsrichtung in gleichen Abstände beziehungsweise gleichen Winkelabständen angeordnet sein, um eine gleichmäßige Flüssigkeitszufuhr aus dem Flüssigkeitstank 30 und/oder den Kammern 30a, 30b zu erlauben. Die Liquidzuführelemente 16a, 16b können in einer Ausführungsform entlang des Strömungskanals 13 angeordnet sein. Der Flüssigkeitstank 30 kann einteilig sein und die durch die Trennwand 43 getrennten Kammern 30a, 30b umfassen. Der Flüssigkeitstank 30 kann mehrteilig sein und getrennten Kammern 30a, 30b können unabhängig voneinander in die Verdampfer-Tank-Einheit 26 einsetzbar sein.

Figur 4 zeigt einen Längsschnitt und Figur 5 einen Querschnitt durch eine radiale Ebene der Verdampfer-Tank-Einheit 26. Der Schnitt verdeutlicht, dass das Grundteil 14 vorzugsweise radial innerhalb des Gehäuses 37 des beziehungsweise Flüssigkeitstanks 30 beziehungsweise der Kammern 30a, 30b angeordnet ist.

Je nach Ausführung kann eine beliebige Anzahl oder Mehrzahl an Verdampfern 3a, 3b in dem Verdampfereinsatz 1 vorgesehen sein, die über ihr jeweiliges Liquidzuführelement 16a, 16b aus einer oder mehreren Kammern 30a, 30b eine oder mehrere Flüssigkeiten 2a, 2b verdampfen können. Eine die Verdampfer 3a, 3b aufweisende standardisierte Verdampferbaugruppe inklusive Trägermaterial kann vorzugsweise sowohl in einen Verdampfereinsatz 1 eingebaut werden, welcher für die Verwendung mit einer Flüssigkeit 2 oder mit mehreren Flüssigkeiten 2a, 2b eingerichtet ist.

Figuren 6 und 7 zeigen eine Verdampfer-Tank-Einheit 26 mit einem eckigen, beispielsweise hexagonalen Querschnitt und einem in dem Montagekanal 35 eingesetzten Verdampfereinsatz 1. Die äußere Form des Flüssigkeitstanks 30 beziehungsweise des Gehäuses 37 ist unabhängig von der Form des Verdampfereinsatzes 1.

Der Verdampfereinsatz 1 wird zur Montage in eine vom Gehäuse 37 des Flüssigkeitstanks 30 gebildete Einsetzöffnung 42 in den Montagekanal 35 eingeführt. In dem Montagekanal 35 ist der Verdampfereinsatz 1 in seiner Betriebsposition verdrehsicher und verschiebefest gehalten. Durch die verdrehsichere und verschiebefeste Halterung des Verdampfereinsatzes 1 in dem Montagekanal 35 kann eine zuverlässige Flüssigkeitszuführung aus dem Flüssigkeitsspeicher 30 und elektrische Kontaktierung mit einem Komponententeil des Inhalators 27 sichergestellt werden.

Der Verdampfereinsatz 1 weist in diesem Ausführungsbeispiel mehrere, hier zwei Halte- und/oder Befestigungselemente 38 auf. Ein Halte- und/oder Befestigungselement 38 ist im Bereich des Kamins 40 angeordnet und als eine Aussparung in dem Grundteil 14 ausgebildet. Die Aussparung kann auch an anderen Positionen an der Mantelseite 31 des Grundteils 14 vorgesehen sein, solange die Innenfläche 36 des Montagekanals 35 eine entsprechende Ausbeulung aufweist, um eine formschlüssige Verbindung zwischen Flüssigkeitstank 30 und Verdampfereinsatz 1 zu erlauben. Das Grundteil 14 weist einen abschnittsweise runden Querschnitt auf, der sich zwischen Kamin 40 und Schlot 41 stromabwärts verjüngt. Vorteilhaft verjüngt sich das Grundteil 14 von der Bodenplatte 7 ausgehend monoton. Die Verjüngung der Mantelseite 31 des Grundteils 14 bewirkt ein Anschlag, der das beliebige Verschieben entlang der Längsachse L des Verdampfereinsatzes 1 beziehungsweise entlang des Montagekanals 35 verhindert. Die Verjüngung kann somit als Halte- und/oder Befestigungselement 38 aufgefasst werden. Der Verdampfereinsatz 1 weist in der gezeigten Ausführungsform eine kranzartige Verbreiterung an dem der Bodenplatte 7 zugeordneten Ende auf.

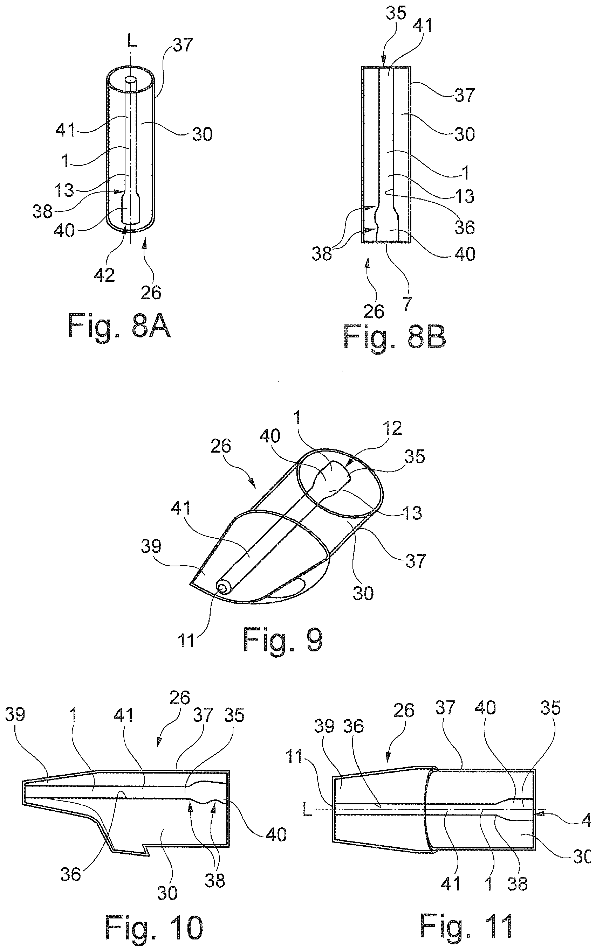

Figur 8 zeigt eine Verdampfer-Tank-Einheit 26 mit einem runden Querschnitt und verdeutlicht unterschiedliche, mögliche und nicht abschließende Formgebungen der Flüssigkeitsspeicher 30 bei gleichbleibender Anbindung und Montage des Verdampfereinsatzes 1.

Figuren 9 bis 11 zeigen eine Verdampfer-Tank-Einheit 26, mit einem Mundende 39 an dem ein Konsument zur Inhalation ziehen kann. In diesem Ausführungsbeispiel erstreckt sich der Schlot 41 beziehungsweise das Grundteil 14 und der Luftkanal 13 bis zum Mundende 39. Die dem Mundende 39 zugeordnete Stirnseite 11 fluchtet mit dem Mundende 39.

In den gezeigten Ausführungsformen umschließt das Grundteil 14 den Luftkanal 13 vollständig. In anderen, nicht gezeigten Ausführungsformen kann der Luftkanal wenigstens abschnittsweise durch das Gehäuse des Flüssigkeitstanks 30 beziehungsweise die Innenfläche 36 des Montagekanals 35 und/oder eine Verlängerung der Innenfläche 36 gebildet werden.

Figur 12 zeigt eine schematische Darstellung eines Inhalators 27. Der Inhalator 27, hier ein elektronisches Zigarettenprodukt, umfasst ein Inhalatorgehäuse 51, in dem ein Luftkanal 52 zwischen mindestens einer Lufteinlassöffnung 57 und einer Luftauslassöffnung 24 an einem Mundende 39 des Zigarettenprodukts 27 vorgesehen ist. Das Mundende 39 des Zigarettenprodukts 27 bezeichnet dabei das Ende, an dem der Konsument zwecks Inhalation zieht und dadurch das Zigarettenprodukt 27 mit einem Unterdruck beaufschlagt und eine Luftströmung 53 in dem Luftkanal 52 erzeugt. Die Luftströmung 53 bedingt einen Luftstrom 5 durch den Strömungskanal 13 des Verdampfereinsatzes 1.

Das Zigarettenprodukt 27 besteht vorteilhaft aus einem Basisteil 69 und einer Verbrauchseinheit 26 mit einem Flüssigkeitstank 30 und einem Verdampfereinsatz 1. Die Verbrauchseinheit kann insbesondere in Form einer auswechselbaren Kartusche ausgebildet sein, oder in Form eines Flüssigkeitstanks 30 in dem der Konsument den Verdampfereinsatz 1 einsetzen kann.

Die durch die Lufteinlassöffnung 57 angesaugte Luft wird in dem Luftkanal 52 zu dem Verdampfereinsatz 1 geleitet. Der Verdampfereinsatz 1 ist mit mindestens einem Flüssigkeitstank 30 verbunden oder verbindbar, in dem mindestens eine Flüssigkeit 2 gespeichert ist. Der Verdampfereinsatz 1 verdampft Flüssigkeit 2, die ihm aus dem Flüssigkeitstank 30 zugeführt wird, und gibt die verdampfte Flüssigkeit 2 als Aerosol/Dampf an einer Auslassseite an einer Stirnseite 11 des Verdampfereinsatzes 1 in den Luftstrom 53 zu. Ein vorteilhaftes Volumen des Flüssigkeitstanks 30 liegt im Bereich zwischen 0,1 ml und 5 ml, vorzugsweise zwischen 0,5 ml und 3 ml, weiter vorzugsweise zwischen 0,7 ml und 2 ml oder 1,5 ml.

Über die Lufteinlassöffnung 57 beziehungsweise eine Luftzuführung beispielsweise an der Lufteinlassöffnung 57 zugewandten Stirnseite 12 des Verdampfereinsatzes 1 kann der Zugwiderstand definiert werden. Bevorzugt ist die Luftzuführung an der Stirnseite 12 beziehungsweise in einer Öffnung einer Bodenplatte 7 des Verdampfereinsatzes 1 vorgesehen. Der Zugwiderstand kann weiter durch entsprechend geformte Löcher beziehungsweise Lufteintrittsöffnungen und Ausformungen im Strömungskanal 13 des Verdampfereinsatzes 1 beeinflusst werden. Es ist jedoch auch denkbar eine Düse oder ein definiertes Lochplättchen als zusätzliches Teil einzufügen.

Die elektronische Zigarette 27 umfasst des Weiteren einen elektrischen Energiespeicher 55 und eine elektronische Steuerungsvorrichtung 56. Der Energiespeicher 55 ist in der Regel in dem Basisteil 69 angeordnet und kann insbesondere eine elektrochemische Einweg-Batterie oder ein wiederaufladbarer elektrochemischer Akku, beispielsweise ein Lithium-Ionen-Akku, sein. Die elektronische Steuerungsvorrichtung 56 umfasst mindestens eine digitale Datenverarbeitungseinrichtung, insbesondere Mikroprozessor und/oder Mikrocontroller, in dem Basisteil 69 und/oder in der Verbrauchseinheit 26.Das Basisteil 69 umfasst zweckmäßigerweise eine elektronische Schnittstelle 70, die bei eingesetzter Verdampfer-Tank-Einheit 26 zur Kontaktierung der elektrischen Kontakte 10 eingerichtet und angeordnet ist. Die elektronische Steuerungsvorrichtung 56 und der Verdampfereinsatz 1 sind somit vorteilhaft über die elektronische Schnittstelle 70 mit den beispielhaft in Figuren 1 bis 4 gezeigten elektrischen Kontakten 10 der Verbrauchseinheit miteinander verbunden beziehungsweise verbindbar.

In dem Inhalatorgehäuse 51 ist vorteilhaft ein Sensor, beispielsweise ein Unterdrucksensor 46 oder ein Druck- oder Strömungsschalter, angeordnet, wobei die Steuerungsvorrichtung 56 auf der Grundlage eines von dem Unterdrucksensor 46 ausgegebenen Sensorsignals feststellen kann, dass ein Konsument am Mundende 29 des Zigarettenprodukts 27 zieht, um zu inhalieren. In diesem Fall ist die in Figuren 1 bis 4 gezeigte elektronische Einheit 4 dazu eingerichtet den Verdampfer 3 anzusteuern, wenn der Unterdrucksensor 46 einen durch Ziehen bedingten Unterdruck misst. Beispielsweise kann der Unterdrucksensor 46 ein Signal an die Steuerungsvorrichtung 56 leiten, um eine Zugaktivierung des Verdampfereinsatzes 1 zu ermöglichen. Die Steuerungsvorrichtung 56 steuert beispielsweise den Verdampfereinsatz 1 an, um Flüssigkeit 2 aus dem Flüssigkeitstank 30 als Aerosol/Dampf in den Luftstrom 53 zuzugeben.

Die in dem Flüssigkeitstank 30 gespeicherte, zu dosierende Flüssigkeit 2 ist beispielsweise eine Mischung aus 1,2-Propylenglykol, Glycerin, Wasser, mindestens einem Aroma (Flavour) und/oder mindestens einem Wirkstoff, insbesondere Nikotin. Andere Wirkstoffe und Flüssigkeitsgemische, beispielsweise für medizinische Anwendungen, sind nicht ausgeschlossen.

Die Verbrauchseinheit bzw. Kartusche umfasst vorteilhaft einen nichtflüchtigen Datenspeicher zum Speichern von der Verbrauchseinheit bzw. Kartusche betreffender Information bzw. Parameter. Der Datenspeicher kann Teil der elektronischen Steuerungsvorrichtung 56 sein. In dem Datenspeicher ist vorteilhaft Information zur Zusammensetzung der in dem Flüssigkeitstank 30 gespeicherten Flüssigkeit, Information zum Prozessprofil, insbesondere Leistungs-/Temperatursteuerung; Daten zur Zustandsüberwachung bzw. Systemprüfung, beispielsweise Dichtigkeitsprüfung; Daten betreffend Kopierschutz und Fälschungssicherheit, eine ID zur eindeutigen Kennzeichnung der Verbrauchseinheit bzw. Kartusche, Seriennummer, Herstelldatum und/oder Ablaufdatum, und/oder Zugzahl (Anzahl der Inhalationszüge durch den Konsumenten) bzw. der Nutzungszeit gespeichert. Der Datenspeicher ist vorteilhaft über Kontakte und/oder Leitungen mit der Steuereinrichtung 56 und/oder der elektronischen Einheit 4 verbunden oder verbindbar.

Der Verdampfereinsatz 1 umfasst vorzugsweise die Luftzuführung, die Luftführung, die Dampfabführung mit möglicher Rekondensationsbehandlung, einer Be- und/oder Entlüftungseinrichtungen 17, Vor- und/oder Nachbehandlungsbereiche 18, eine eindeutigen elektronischen Identifizierung und/oder elektrischen Kontakten 10 standardmäßig, damit alle wichtigen Funktionen einer Kartusche für den Inhalator 27 erfüllt werden, die in möglichst vielseitig geformte Flüssigkeitstanks 30 integriert werden kann.

Figur 13 zeigt eine schematische Darstellung einer beispielhaften Ausführungsform eines Verdampfers 3.

Der beispielhafte Verdampfer 3 gemäß Figur 13 umfasst einen blockförmigen, vorzugsweise monolithischen Heizkörper 60 aus einem elektrisch leitenden Material. Der Heizkörper 60 kann auch eine durch zum Beispiel Laserbearbeitung perforierte Struktur aufweisen.

Der Heizkörper 60 ist mit einer Mehrzahl von Mikrokanälen 62 versehen, die eine Einlassseite 61 des Heizkörpers 60 mit einer Auslassseite 64 flüssigkeitsleitend verbinden. Die Einlassseite 61 ist über eine Dochtstruktur beziehungsweise ein Liquidzuführelement 16 flüssigkeitsleitend mit dem Flüssigkeitstank 30 verbunden. Das Liquidzuführelement 16 dient zur passiven Förderung von Flüssigkeit 2 aus einem Flüssigkeitstank 30 zu dem Heizkörper 60 mittels Kapillarkräften, um für eine zuverlässige Aerosolproduktion durch den bzw. die Verdampfer 3 zu jedem Zeitpunkt eine ausreichende Flüssigkeitsversorgung bereitzustellen. Im Kontaktbereich beziehungsweise der Einlassseite 61 zu dem Heizkörper 60 dient das Liquidzuführelement 16 dazu, Flüssigkeit 2 gleichmäßig zu verteilen, temperaturbeständig zu sein und mit ihren relativ kleinen Poren und/oder dünnen Kapillaren eine Art Rückschlagventil zu bilden, um unerwünschtes Rückfließen von blasenhaltiger Flüssigkeit 2 aus dem Heizkörper 60 in das Liquidzuführelement 16 und/oder in den Flüssigkeitstank 30 zu verhindern.

Der mittlere Durchmesser der Mikrokanäle 62 liegt vorzugsweise im Bereich zwischen 5 µm und 200 µm, weiter vorzugsweise im Bereich zwischen 30 µm und 150 µm, noch weiter vorzugsweise im Bereich zwischen 50 µm und 100 µm. Aufgrund dieser Abmessungen wird vorteilhaft eine Kapillarwirkung erzeugt, so dass an der Einlassseite 61 in einen Mikrokanal 62 eindringende Flüssigkeit durch den Mikrokanal 62 nach oben beziehungsweise bis zur Auslassseite 64 steigt, bis der Mikrokanal 62 mit Flüssigkeit gefüllt ist. Das Volumenverhältnis von Mikrokanälen 62 zu Heizkörper 60, das als Porosität des Heizkörpers 60 bezeichnet werden kann, liegt beispielsweise im Bereich zwischen 10% und 50%, vorteilhaft im Bereich zwischen 15% und 40%, noch weiter vorteilhaft im Bereich zwischen 20% und 30%, und beträgt beispielsweise 25%.

Die Kantenlängen der mit Mikrokanälen 62 versehenen Flächen des Heizkörpers 60 liegen beispielsweise im Bereich zwischen 0,5 mm und 3 mm, vorzugsweise zwischen 0.5 mm und 1 mm. Die Abmessungen der mit Mikrokanälen 62 versehenen Flächen des Heizkörpers 60 können beispielsweise betragen: 0,95 mm x 1,75 mm oder 1,9 mm x 1,75 mm oder 1,9 mm x 0,75 mm. Die Kantenlängen des Heizkörpers 60 können beispielsweise im Bereich zwischen 0,5 mm und 5 mm, vorzugsweise im Bereich zwischen 0,75 mm und 4 mm, weiter vorzugsweise im Bereich zwischen 1 mm und 3 mm liegen. Die Fläche des Heizkörpers 60 (chip size) kann beispielsweise 1 mm x 3 mm, 2 mm x 2 mm oder 2 mm x 3 mm betragen.

Die Breite b des Heizkörpers 60 (siehe Figur 13) liegt vorzugsweise im Bereich zwischen 1 mm und 5 mm, weiter vorzugsweise im Bereich zwischen 2 mm und 4 mm, und beträgt beispielsweise 3 mm. Die Höhe h des Heizkörpers 60 (siehe Figur 13) liegt vorzugsweise im Bereich zwischen 0,05 mm und 1 mm, weiter vorzugsweise im Bereich zwischen 0,1 mm und 0,75 mm, noch weiter vorzugsweise im Bereich zwischen 0,2 mm und 0,5 mm und beträgt beispielsweise 0,3 mm.

Die Anzahl der Mikrokanäle 62 liegt vorzugsweise im Bereich zwischen vier und 1000. Auf diese Weise lässt sich der Wärmeeintrag in die Mikrokanäle 62 optimieren und eine gesicherte hohe Verdampfungsleistung sowie eine ausreichend große Dampfaustrittsfläche realisieren.

Die Mikrokanäle 62 sind in Form eines quadratischen, rechteckigen, vieleckigen, runden, ovalen oder anders geformten Arrays angeordnet. Das Array kann in Form einer Matrix mit s Spalten und z Zeilen ausgebildet sein, wobei s vorteilhaft im Bereich zwischen 2 und 50 und weiter vorteilhaft im Bereich zwischen 3 und 30 und/oder z vorteilhaft im Bereich zwischen 2 und 50 und weiter vorteilhaft im Bereich zwischen 3 und 30 liegt. Auf diese Weise lässt sich eine effektive und auf einfache Weise herstellbare Anordnung der Mikrokanäle 62 mit gesichert hoher Verdampfungsleistung realisieren.

Der Querschnitt der Mikrokanäle 62 kann quadratisch, rechteckig, vieleckig, rund, oval oder anders geformt sein, und/oder sich in Längsrichtung abschnittweise ändern, insbesondere vergrößern, verkleinern oder konstant bleiben. Die Mikrokanäle 62 können auch bionische Strukturen mit einer besonders großen Oberfläche aufweisen.

Die Länge eines oder jedes Mikrokanals 62 liegt vorzugsweise im Bereich zwischen 100 µm und 1000 µm, weiter vorzugsweise im Bereich zwischen 150 µm und 750 µm, noch weiter vorzugsweise im Bereich zwischen 180 µm und 500 µm und beträgt beispielsweise 300 µm. Auf diese Weise lässt sich eine optimale Flüssigkeitsaufnahme und Portionsbildung bei ausreichend gutem Wärmeeintrag von dem Heizkörper 60 in die Mikrokanäle 62 realisieren.

Der Abstand zweier Mikrokanäle 62 beträgt vorzugsweise mindestens das 1,3-fache des lichten Durchmessers eines Mikrokanals 62, wobei der Abstand auf die Mittelachsen der beiden Mikrokanäle 62 bezogen ist. Der Abstand kann bevorzugt das 1,5- bis 5-fache, weiter bevorzugt das 2- bis 4-fache des lichten Durchmessers eines Mikrokanals 62 betragen. Auf diese Weise lässt sich ein optimaler Wärmeeintrag in die Mikrokanäle und eine ausreichend stabile Anordnung und Wandstärke der Mikrokanäle realisieren.

Der Verdampfereinsatz 1 weist eine vorzugsweise von der Steuerungsvorrichtung 56 steuerbare Heizspannungsquelle 71 auf, die über Elektroden 72 an gegenüberliegenden Seiten des Heizkörpers 60 mit diesem verbunden ist, so dass eine von der Heizspannungsquelle 71 erzeugte elektrische Spannung Uh zu einem Stromfluss durch den Heizkörper 60 führt. Aufgrund des Ohm'schen Widerstands des elektrisch leitenden Heizkörpers 60 führt der Stromfluss zu einer Erhitzung des Heizkörpers 60 (Joule'sche Wärme) und daher zu einer Verdampfung von in den Mikrokanälen 62 enthaltener Flüssigkeit. Der Heizkörper 60 wirkt somit als Verdampfer. Auf diese Weise erzeugter Dampf/Aerosol entweicht zur Auslassseite 64 aus den Mikrokanälen 62 und wird dem Luftstrom 5 im Strömungskanal 13 beigemischt, siehe Figur 1. Genauer steuert bei Feststellung eines durch Ziehen des Konsumenten verursachten Luftstroms 5 durch den Strömungskanal 13 die Steuerungsvorrichtung 56 die Heizspannungsquelle 71 an, wobei durch spontane Erhitzung die in den Mikrokanälen 62 befindliche Flüssigkeit in Form von Dampf/Aerosol aus den Mikrokanälen 62 getrieben wird.

Dabei kann die Dauer der einzelnen Verdampfungsschritte bei unterschiedlichen Temperaturen und/oder einem Verdampfen der einzelnen Komponenten der einzelnen Portionen der Flüssigkeit derart kurz gehalten werden und/oder mit einer Ansteuerfrequenz getaktet erfolgen, dass die schrittweise Verdampfung von einem Konsumenten nicht wahrgenommen und trotzdem eine weitgehend homogene, geschmackskonforme, wiederholbar präzise Aerosolbildung gewährleistet werden kann. Insbesondere erfolgt vorteilhaft zunächst ein Verdampfen einer leichter siedenden Komponente der Flüssigkeit in einem ersten Verdampfungsintervall mit einer ersten Temperatur A und anschließend ein Verdampfen einer höher siedenden Komponente der Flüssigkeit in einem zweiten Verdampfungsintervall mit einer zweiten Temperatur B, welche die Temperatur A übersteigt.

Vorzugsweise ist in dem Datenspeicher des Inhalators 27 eine dem verwendeten Flüssigkeitsgemisch angepasste Spannungskurve Uh(t) hinterlegt. Dies ermöglicht es, den Spannungsverlauf Uh(t) dem verwendeten Liquid angepasst vorzugeben, so dass sich die Heiztemperatur des Heizkörpers 60, und damit auch die Temperatur der kapillaren Mikrokanäle 62, gemäß der bekannten Verdampfungskinetik des jeweiligen Liquids zeitlich über den Verdampfungsvorgang steuern lässt, wodurch optimale Verdampfungsergebnisse erzielbar sind. Die Verdampfungstemperatur liegt vorzugsweise im Bereich zwischen 100°C und 400°C, weiter bevorzugt zwischen 150 C und 350°C, noch weiter bevorzugt zwischen 190 C und 290 C.

An der Einlassseite 61 des Heizkörpers 60 ist eine poröse und/oder kapillare, flüssigkeitsleitende Dochtstruktur beziehungsweise das Liquidzuführelement 16 angeordnet. Das Liquidzuführelement 16 kontaktiert die Einlassseite 61 des Heizkörpers 60 flächig und deckt sämtliche Mikrokanäle 62 einlassseitig ab, wie in den Figur 13 ersichtlich ist. An der dem Heizkörper 60 gegenüberliegenden Seite ist die das Liquidzuführelement 16 flüssigkeitsleitend mit dem Flüssigkeitstank 30 verbunden. Die in den Figuren 12 und 13 gezeigte direkte Anbindung des Flüssigkeitstanks 30 an das Liquidzuführelement 16 ist nur beispielhaft zu verstehen. Insbesondere können eine Flüssigkeitsschnittstelle und/oder eine mehrere Flüssigkeitsleitungen zwischen Flüssigkeitstank 30 und dem Liquidzuführelement 16 vorgesehen sein. Der Flüssigkeitstank 30 kann daher auch beabstandet von dem Liquidzuführelement 16 angeordnet sein. Der Flüssigkeitstank 30 kann in seinen Abmessungen größer als das Liquidzuführelement 16 sein. Das Liquidzuführelement 16 kann beispielsweise in eine Zuführöffnung 45 eines Gehäuses 37 des Flüssigkeitstanks 30 eingesetzt sein. Es kann auch eine Mehrzahl von Verdampfern 3 einem Flüssigkeitstank 30 zugeordnet sein.

Das Liquidzuführelement 16 kann in dem Grundteil 14 und/oder der Bodenplatte 7 des Verdampfereinsatzes 1 angeordnet sein. Das Liquidzuführelement 16 kann generell einteilig oder mehrteilig sein.

Das Liquidzuführelement 16 besteht aus porösem und/oder kapillarem Material, das aufgrund von Kapillarkräften in der Lage ist, von dem Heizkörper 60 verdampfte Flüssigkeit in ausreichender Menge von dem Flüssigkeitstank 30 zu dem Heizkörper 60 passiv nachzufördern, um ein Leerlaufen der Mikrokanäle 62 und sich daraus ergebende Probleme zu verhindern.

Das Liquidzuführelement 16 besteht vorteilhaft aus einem nichtleitenden Material, um eine unerwünschte Erwärmung von Flüssigkeit in dem Liquidzuführelement 16 durch elektrischen Stromfluss zu vermeiden. Falls das Liquidzuführelement 16 aus einem leitenden Material besteht, was nicht ausgeschlossen ist, ist zwischen der dem Liquidzuführelement 16 und dem Heizkörper 60 vorteilhaft eine Isolierschicht aus einem elektrisch und/oder thermisch isolierenden Material, beispielsweise Glas, Keramik oder Kunststoff, mit sich durch die Isolierschicht erstreckenden, mit den Mikrokanälen 62 korrespondierenden Durchgangsöffnungen vorgesehen.

Das Liquidzuführelement 16 besteht vorteilhaft aus einem oder mehreren der Materialien Baumwolle, Cellulose, Acetat, Glasfasergewebe, Glasfaserkeramik, Sinterkeramik, keramisches Papier, Alumosilikat-Papier, Metallschaum, Metallschwamm, einem anderen hitzebeständigen, porösen und/oder kapillaren Material mit geeigneter Förderrate, oder einem Verbund von zwei oder mehreren der vorgenannten Materialien. Das Liquidzuführelement 16 kann in einer Ausführungsform ein klassischer Docht sein. In einer vorteilhaften praktischen Ausführungsform kann das Liquidzuführelement 16 mindestens ein Keramikfaserpapier und/oder eine poröse Keramik umfassen. Das Volumen der Dochtstruktur 19 liegt vorzugsweise im Bereich zwischen 1 mm³ und 10 mm³, weiter vorzugsweise im Bereich zwischen 2 mm³ und 8 mm³, noch weiter vorzugsweise im Bereich zwischen 3 mm³ und 7 mm³ und beträgt beispielsweise 5 mm³.

Oberflächen des Heizkörpers 60 können vorteilhaft hydrophil sein. Die Einlassseite 61 und/oder die Auslassseite 64 des Heizkörpers 60 kann vorteilhaft mikrostrukturiert sein bzw. Mikroausnehmungen (micro grooves) aufweisen.

Der Verdampfereinsatz 1 ist so eingestellt, dass eine Flüssigkeitsmenge vorzugsweise im Bereich zwischen 1 µl und 20 µl, weiter vorzugsweise zwischen 2 µl und 10 µl, noch weiter vorzugsweise zwischen 3 µl und 5 µl, typischerweise 4 µl pro Zug des Konsumenten, zudosiert wird. Vorzugsweise kann der Verdampfereinsatz 1 hinsichtlich der Flüssigkeits-/Dampfmenge pro Zug einstellbar sein.

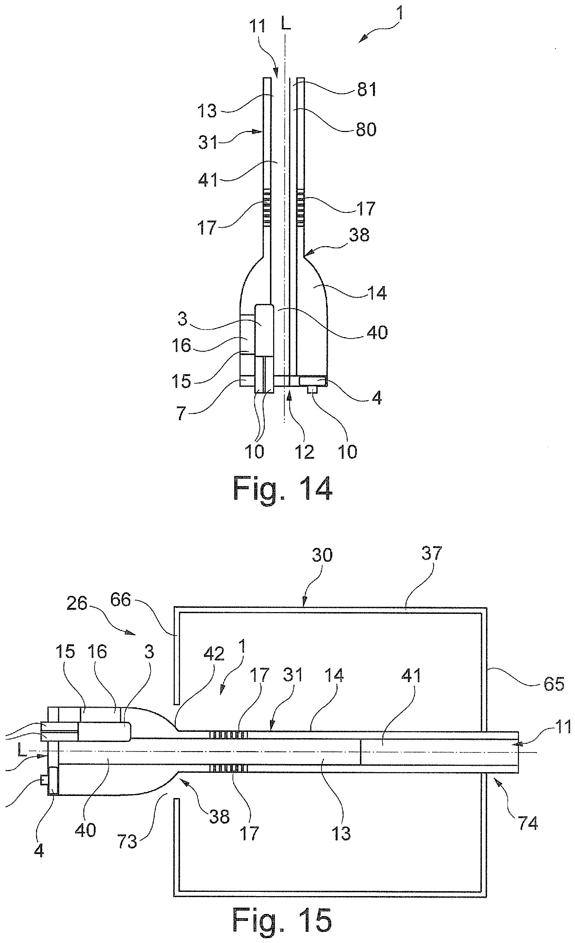

Figur 14 zeigt einen Längsschnitt durch einen Verdampfereinsatz 1 mit einem Nebenluftkanal 80. Der Verdampfereinsatz 1 umfasst eine Separationswand 81 und die Separationswand 80 trennt den Strömungskanal 13 vom Nebenluftkanal 80. Die Separationswand 80 verläuft vorzugsweise abschnittsweise parallel zur Längsachse L des Verdampfereinsatzes 1. Die Separationswand 81 kann beispielsweise röhrenförmig beziehungsweise hohlzylinderförmig sein und/oder mit einer Innenwand des Grundteil 13 verbunden sein. Die Separationswand 81 und/oder der Nebenluftkanal 80 erstreckt sich vorteilhaft durch den gesamten Verdampfereinsatz 1. Vorteilhaft ist ein durch den Nebenluftkanal 80 führbarer Nebenluftstrom von einem im Inhalator 27 vorgesehenen Unterdrucksensor 46 sensierbar.

Figur 15 zeigt eine fertigungstechnisch vorteilhafte Verdampfer-Tank-Einheit 26. Die Verdampfer-Tank-Einheit 26 weist in diesem Beispiel ein Gehäuse 37 mit einer Einsetzöffnung 42 und einer Ausgangsöffnung 74, jedoch keine Innenwand 36 und keinen Montagekanal 35 auf.

Zur Bereitstellung der Verdampfer-Tank-Einheit 26 wird der Verdampfereinsatz 1 mit der auslassseitigen Stirnseite 11 zu der Ausgangsöffnung 74 geführt, insbesondere geschoben, damit das Grundteil 14 beziehungsweise die Mantelseite 31 die Ausgangsöffnung 74 flüssigkeitsdicht verschließt. Durch die Formgebung des Grundteils 14 ergibt sich eine zwischen dem Grundteil 14 und der Einsetzöffnung 42 angeordnete Befüllöffnung 73. Über die Befüllöffnung 73 kann der Flüssigkeitstank 30 mit in der Abbildung nicht dargestellter Flüssigkeit befüllt werden. Nach dem Befüllen des Flüssigkeitstanks 30 wird der Verdampfereinsatz 1 bis zu einem Anschlag durch die Einsetzöffnung 42 geführt, insbesondere geschoben, damit das Grundteil 14 beziehungsweise die Mantelseite 31 die Einsetzöffnung 42 flüssigkeitsdicht verschließt und so eine befüllte und leckagefreie Verdampfer-Tank-Einheit 26 bereitstellt.

Im montierten Zustand der Verdampfer-Tank-Einheit 26 erstreckt sich der Verdampfereinsatz 1 durch die Einsetzöffnung 42 bis zur Ausgangsöffnung 74. In diesem Beispiel ragt der Schlot 41 des Verdampfereinsatzes 1 aus der Ausgangsöffnung 74 heraus. Vorzugsweise wird der Schlot 74 gestutzt, um mit der Stirnseite 65 des Gehäuses 37 beziehungsweise des Flüssigkeitstanks 30 zu fluchten.

Die zuvor geschilderte Befüllung für den Flüssigkeitstank 30 ist nicht obligatorisch. Alternativ kann beispielsweise die Verdampfer-Tank-Einheit 26 fertig montiert und der Tank 30 über eine entsprechende Befüllöffnung befüllbar sein. Andere Befüllarten sind möglich.

## Patentansprüche

1. Verdampfer-Tank-Einheit (26) umfassend einen Verdampfereinsatz (1) für einen Inhalator (27) umfassend
- mindestens einen elektrischen Verdampfer (3) zum Verdampfen von dem Verdampfer (3) zugeführter Flüssigkeit (2), und
- elektrische Kontakte (10) zur elektrischen Kontaktierung des Verdampfereinsatzes (1) für die Versorgung mit elektrischer Energie und/oder zum Empfangen von Steuersignalen für den Verdampfer (3), wobei
- der Verdampfereinsatz (1) ein Grundteil (14) mit gegenüberliegend angeordneten Stirnseiten (11, 12) und einer um mindestens einen durchströmbaren Strömungskanal (13) angeordneten Mantelseite (31) zwischen den Stirnseiten (11, 12) aufweist, wobei
- an der Mantelseite (31) des Grundteils (14) mindestens eine Flüssigkeitsöffnung (15) zum Zuführen von verdampfbarer Flüssigkeit (2) von außen in den Verdampfereinsatz (1) zu dem Verdampfer (3) angeordnet ist, wobei
- die Verdampfer-Tank-Einheit (26) einen Flüssigkeitstank (30) umfasst, in dem der Verdampfereinsatz (1) eingesetzt ist, wobei
- der Flüssigkeitstank (30) eine Einsetzöffnung (42) und eine Ausgangsöffnung (74) aufweist, wobei
- sich der Verdampfereinsatz (1) von der Einsetzöffnung (42) bis zur Ausgangsöffnung (74) erstreckt,
**dadurch gekennzeichnet, dass**
der insbesondere patronen- oder hülsenförmige Verdampfereinsatz (1) in einem externen Teil, nämlich in dem Flüssigkeitstank (30), anordenbar und in dieses einschiebbar ist, wobei der Verdampfereinsatz (1) verdrehsicher in der Verdampfer-Tank-Einheit (26) gehalten ist, wobei
- der Verdampfer (3) einen blockförmigen Heizkörper (60) aus einem elektrisch leitenden Material umfasst, wobei
- der Heizkörper (60) mit einer Mehrzahl von Mikrokanälen (62) versehen ist, die eine Einlassseite (61) des Heizkörpers (60) mit einer Auslassseite (64) flüssigkeitsleitend verbinden, wobei
- die Einlassseite (61) über eine Dochtstruktur oder ein Liquidzuführelement (16) flüssigkeitsleitend mit dem Flüssigkeitstank (30) verbunden ist, wobei
- das Liquidzuführelement (16) zur passiven Förderung von Flüssigkeit (2) aus einem Flüssigkeitstank (30) zu dem Heizkörper (60) mittels Kapillarkräften dient.

2. Verdampfer-Tank-Einheit (26) nach Anspruch 1, **dadurch gekennzeichnet, dass**
- der Verdampfereinsatz (1) mindestens ein Halte- und/oder Befestigungselement (38) zur Halterung und/oder Befestigung des Verdampfereinsatzes (1) in dem externen Teil (30) umfasst.

3. Verdampfer-Tank-Einheit (26) nach Anspruch 1 oder 2,
**dadurch gekennzeichnet, dass**
- der Verdampfereinsatz (1) in das externe Teil (30) verdrehsicher einbaubar ist.

4. Verdampfer-Tank-Einheit (26) nach einem der vorangehenden Ansprüche, **dadurch gekennzeichnet, dass**
- die elektrischen Kontakte (10) an einer der Stirnseiten (11, 12) des Verdampfereinsatzes (1) angeordnet sind.

5. Verdampfer-Tank-Einheit (26) nach einem der vorangehenden Ansprüche, **dadurch gekennzeichnet, dass**
- an der Mantelseite des Grundteils (14) mindestens eine luftdurchlässige Be- und/oder Entlüftungseinrichtung (17) vorgesehen ist.

6. Verdampfer-Tank-Einheit (26) nach einem der vorangehenden Ansprüche, **dadurch gekennzeichnet, dass**
- der Strömungskanal (13) mindestens einen Vor- und/oder Nachbehandlungsbereich (18) zur Vor- und/oder Nachbehandlung durch den Strömungskanal (13) strömender Luft und/oder verdampfter Flüssigkeit (2) umfasst.

7. Verdampfer-Tank-Einheit (26) nach einem der vorangehenden Ansprüche, **dadurch gekennzeichnet, dass**
- der Verdampfereinsatz (1) eine Mehrzahl von elektrischen Verdampfern (3a, 3b) umfasst.

8. Verdampfer-Tank-Einheit (26) nach Anspruch 7, **dadurch gekennzeichnet, dass**
- die elektrischen Verdampfer (3a, 3b) entlang des Umfangs der Mantelseite (31) des Grundteils (14) und/oder entlang des Strömungskanals (13) angeordnet sind.

9. Verdampfer-Tank-Einheit (26) nach einem der Ansprüche 7 oder 8, dass
- der Verdampfereinsatz (1) eine Mehrzahl von Flüssigkeitsöffnung (15a, 15b) aufweist, wobei
- jedem elektrischen Verdampfer (3a, 3b) Flüssigkeit (2a, 2b) aus einer entsprechenden Flüssigkeitsöffnung (15a, 15b) zuführbar ist.

10. Verdampfer-Tank-Einheit (26) nach einem der vorangehenden Ansprüche, **dadurch gekennzeichnet, dass**
- der Verdampfereinsatz (1) an der Mantelseite (31) des Grundteils (14) wenigstens ein Halteelement, insbesondere einen Dorn (32) zur Halterung eines kapillaren Elements (33) in einem Flüssigkeitstank (30) aufweist.

11. Verdampfer-Tank-Einheit (26) nach einem der vorangehenden Ansprüche, **dadurch gekennzeichnet, dass**
- der Verdampfereinsatz (1) wenigstens einen vom Strömungskanal (13) separaten Nebenluftkanal (80) umfasst.

12. Verdampfer-Tank-Einheit (26) nach einem der vorangehenden Ansprüche, **dadurch gekennzeichnet, dass**
- die Flüssigkeitsöffnung (15) mit einem Liquidzuführelement (16) kommuniziert, wobei
- das Liquidzuführelement (16) dazu eingerichtet ist Flüssigkeit (2) zum Verdampfer (3) zu transportieren.

13. Verdampfer-Tank-Einheit (26) nach einem der vorangehenden Ansprüche, **dadurch gekennzeichnet, dass**
- der Verdampfereinsatz (1) eine elektronische Einheit (4) zum Steuern und/oder Regeln des Verdampfers (3) und/oder zur Speicherung von Kenndaten umfasst.

14. Verdampfer-Tank-Einheit (26) nach einem der vorangehenden Ansprüche, **dadurch gekennzeichnet, dass** das Grundteil (14) patronen- oder hülsenförmig ist.

15. Verdampfer-Tank-Einheit (26) nach einem der vorangehenden Ansprüche, **dadurch gekennzeichnet, dass**
- der Flüssigkeitstank (30) die Mantelseite (31) des Verdampfereinsatzes (1) wenigstens in dem Abschnitt der Flüssigkeitsöffnung (15) umschließt.

16. Verdampfer-Tank-Einheit (26) nach einem der vorangehenden Ansprüche, **dadurch gekennzeichnet, dass**
- der Flüssigkeitsöffnung (15) ein kapillares Element (33) innerhalb des Flüssigkeitstanks (30) zugeordnet ist.

17. Verdampfer-Tank-Einheit (26) nach einem der vorangehenden Ansprüche, **dadurch gekennzeichnet, dass**
- der Flüssigkeitstank (30) eine Mehrzahl voneinander separater Kammern (30a, 30b) umfasst, und
- jedem elektrischen Verdampfer (3a, 3b) Flüssigkeit (2a, 2b) aus einer entsprechenden Kammer (30a, 30b) zuführbar ist.

18. Verdampfer-Tank-Einheit (26) nach einem der vorangehenden Ansprüche, **dadurch gekennzeichnet, dass**
- wenigstens eine der Stirnseiten (11, 12) des Verdampfereinsatzes (1) mit einer entsprechenden Stirnseite (65, 66) des Flüssigkeitstanks (30) fluchtet.

19. Verdampfer-Tank-Einheit (26) nach einem der vorangehenden Ansprüche, **dadurch gekennzeichnet, dass**
- der Flüssigkeitstank (30) mindestens ein luftdurchlässiges Be- und/oder Entlüftungselement (47) aufweist.

20. Inhalator (27) umfassend eine Verdampfer-Tank-Einheit (26) nach einem der vorangehenden Ansprüche, und einen Energiespeicher (55) zur elektrischen Versorgung des mindestens eines Verdampfers (3).

21. Verfahren zur Fertigung einer Verdampfer-Tank-Einheit (26) nach einem der Ansprüche 1 bis 19 und/oder eines Inhalators (27) nach Anspruch 20, umfassend die Bereitstellung des Verdampfereinsatzes (1) und des Flüssigkeitstanks (30), **dadurch gekennzeichnet, dass** der Verdampfereinsatz (1) in die Einsetzöffnung (42) und bis zu der Ausgangsöffnung (74) des Flüssigkeitstanks (30) eingesetzt, nämlich eingeschoben wird.

22. Verfahren nach Anspruch 21, **dadurch gekennzeichnet, dass**
- der Verdampfereinsatz (1) mit der auslassseitigen Stirnseite (11) bis zur Ausgangsöffnung (74) geführt, nämlich geschoben, wird;
- der Flüssigkeitstank (30) über eine sich zwischen dem Grundteil (14) und der Einsetzöffnung (42) angeordnete oder verbleibende Befüllöffnung (73) mit Flüssigkeit (2) befüllt wird; und
- der Verdampfereinsatz (1) unter Schließung der Befüllöffnung (73) bis zu einem Anschlag durch die Einsetzöffnung (42) geführt, nämlich geschoben, wird.

23. Verfahren nach einem der Ansprüche 21 oder 22, **dadurch gekennzeichnet, dass** der Verdampfereinsatz (1) als Modul gefertigt wird, das einem Fertigungsstrom für eine Verdampfer-Tank-Einheit (26) und/oder einen Inhalator (27) zuführbar ist oder zugeführt wird.

## Claims

1. Vaporiser-tank-unit (26) comprising a vaporiser insert (1) for an inhaler (27) comprising
- at least one electrical vaporiser (3) for vaporising liquid (2) fed to the vaporiser (3), and
- electrical contacts (10) for making electrical contact with the vaporiser insert (1) for supplying with electrical energy and/or for receiving control signals for the vaporiser (3), wherein
- the vaporiser insert (1) has a base part (14) with opposingly arranged faces (11, 12) and a jacket side (31) between the faces (11, 12) arranged around at least one flow channel (13) through which vapour can flow, wherein
- at least one liquid opening (15) for feeding vaporisable liquid (2) from the outside into the vaporiser insert (1) to the vaporiser (3) is arranged on the jacket side (31) of the base part (14), wherein
- the vaporiser-tank-unit (26) comprises a liquid tank (30) in which the vaporiser insert (1) is inserted, wherein
- the liquid tank (30) has an insertion opening (42) and an outlet opening (74), wherein
- the vaporiser insert (1) extends from the insertion opening (42) to the outlet opening (74), **characterised in that**
the in particular cartridge or jacket-shaped vaporiser insert (1) can be arranged in an external part, namely in the liquid tank (30), and slid thereinto, wherein the vaporiser insert (1) is retained in the vaporiser-tank-unit (26) so that it cannot rotate, wherein
- the vaporiser (3) comprises a block-shaped heating element (60) made of an electrically conductive material, wherein
- the heating element (60) is provided with a plurality of microchannels (62), which connect an inlet side (61) of the heating element (60) to an outlet side (64) in a liquid-conducting manner, wherein
- the inlet side (61) is connected via a wick structure or a liquid feed element (16) to the liquid tank (30) in a liquid-conducting manner, wherein
- the liquid feed element (16) serves to passively convey liquid (2) from a liquid tank (30) to the heating element (60) by means of capillary forces.

2. Vaporiser-tank-unit (26) according to claim 1, **characterised in that**
- the vaporiser insert (1) comprises at least one retaining and/or fixing element (38) for retaining and/or fixing the vaporiser insert (1) in the external part (30).

3. Vaporiser-tank-unit (26) according to claim 1 or 2, **characterised in that**
- the vaporiser insert (1) can be fitted in the external part (30) such that it cannot rotate.

4. Vaporiser-tank-unit (26) according to one of the preceding claims, **characterised in that**
- the electrical contacts (10) are arranged on one of the faces (11, 12) of the vaporiser insert (1).

5. Vaporiser-tank-unit (26) according to one of the preceding claims, **characterised in that**
- at least one permeable-to-air ventilation and/or venting device (17) is provided on the jacket side of the base part (14).

6. Vaporiser-tank-unit (26) according to one of the preceding claims, **characterised in that**
- the flow channel (13) comprises at least one pre and/or post-treatment area (18) for pre-treatment and/or post-treatment of air and/or vaporised liquid (2) flowing through the flow channel (13).

7. Vaporiser-tank-unit (26) according to one of the preceding claims, **characterised in that**
the vaporiser insert (1) comprises a plurality of electrical vaporisers (3a, 3b).

8. Vaporiser-tank-unit (26) according to claim 7, **characterised in that**
- the electrical vaporisers (3a, 3b) are arranged along the periphery of the jacket side (31) of the base part (14) and/or along the flow channel (13).

9. Vaporiser-tank-unit (26) according to one of claims 7 or 8, that
- the vaporiser insert (1) has a plurality of liquid openings (15a, 15b), wherein
- liquid (2a, 2b) from a corresponding liquid opening (15a, 15b) can be fed to each electrical vaporiser (3a, 3b).

10. Vaporiser-tank-unit (26) according to one of the preceding claims, **characterised in that**
- the vaporiser insert (1) has at least one retaining element, in particular a spur (32), on the jacket side (31) of the base part (14) for retaining a capillary element (33) in a liquid tank (30).

11. Vaporiser-tank-unit (26) according to one of the preceding claims, **characterised in that** the vaporiser insert (1) comprises at least one secondary air channel (80) which is separate from the flow channel (13).

12. Vaporiser-tank-unit (26) according to one of the preceding claims, **characterised in that**
- the liquid opening (15) communicates with a liquid feed element (16), wherein
- the liquid feed element (16) is designed to transport liquid (2) to the vaporiser (3).

13. Vaporiser-tank-unit (26) according to one of the preceding claims, **characterised in that**
- the vaporiser insert (1) comprises an electronic unit (4) for controlling and/or regulating the vaporiser (3) and/or for storing characteristic data.

14. Vaporiser-tank-unit (26) according to one of the preceding claims, **characterised in that** the base part (14) is cartridge or sleeve-shaped.

15. Vaporiser-tank-unit (26) according to one of the preceding claims, **characterised in that**
- the liquid tank (30) encompasses the jacket side (31) of the vaporiser insert (1) at least in the section of the liquid opening (15).

16. Vaporiser-tank-unit (26) according to one of the preceding claims, **characterised in that**
- a capillary element (33) inside the liquid tank (30) is associated with the liquid opening (15).

17. Vaporiser-tank-unit (26) according to one of the preceding claims, **characterised in that**
- the liquid tank (30) comprises a plurality of chambers (30a, 30b) which are separate from one another, and
- liquid (2a, 2b) from a corresponding chamber (30a, 30b) can be fed to each electrical vaporiser (3a, 3b).

18. Vaporiser-tank-unit (26) according to one of the preceding claims, **characterised in that**
- at least one of the faces (11, 12) of the vaporiser insert (1) is flush with a corresponding face (65, 66) of the liquid tank (30).

19. Vaporiser-tank-unit (26) according to one of the preceding claims, **characterised in that**
- the liquid tank (30) has at least one air-permeable ventilation and/or venting element (47).

20. Inhaler (27) comprising a vaporiser-tank-unit (26) according to one of the preceding claims, and an energy store (55) for the electrical supply of the at least one vaporiser (3).

21. Method for the production of a vaporiser-tank-unit (26) according to one of claims 1 to 19 and/or an inhaler (27) according to claim 20 comprising the provision of the vaporiser insert (1) and the liquid tank (30), **characterised in that** the vaporiser insert (1) is inserted, namely slid, into the insertion opening (42) and as far as the outlet opening (74) of the liquid tank (30).

22. Method according to claim 21, **characterised in that**
- the vaporiser insert (1) is fed, namely slid, with the outlet side face (11) as far as the outlet opening (74);
- the liquid tank (30) is filled with liquid (2) via a filling opening (73) arranged or remaining between the base part (14) and the insertion opening (42); and
- the vaporiser insert (1) is fed, namely slid, through the insertion opening (42) as far as a stop, at the same time closing the filling opening (73).

23. Method according to one of claims 21 or 22, **characterised in that**
the vaporiser insert (1) is produced as a module which can be or is fed to a production flow for a vaporiser-tank-unit (26) and/or an inhaler (27).

## Revendications

1. Unité évaporateur-réservoir (26) comprenant un insert évaporateur (1) pour un inhalateur (27) comprenant
- au moins un évaporateur électrique (3) pour l'évaporation d'un liquide (2) amené à l'évaporateur (3), et
- des contacts électriques (10) pour la mise en contact électrique de l'insert évaporateur (1) pour l'alimentation en énergie électrique et/ou pour la réception de signaux de commande pour l'évaporateur (3), dans laquelle
- l'insert évaporateur (1) présente une partie de base (14) avec des faces d'extrémité (11, 12) disposées de manière opposée et une face d'enveloppe (31) disposée entre les faces d'extrémité (11, 12) autour d'au moins un canal d'écoulement (13) pouvant être traversé par un flux, dans laquelle
- au moins une ouverture pour liquide (15) pour l'amenée d'un liquide (2) évaporable de l'extérieur dans l'insert évaporateur (1) vers l'évaporateur (3) est disposée sur la face d'enveloppe (31) de la partie de base (14), dans laquelle
- l'unité évaporateur-réservoir (26) comprend un réservoir de liquide (30) dans lequel l'insert évaporateur (1) est inséré, dans laquelle
- le réservoir de liquide (30) présente une ouverture d'insertion (42) et une ouverture de sortie (74), dans laquelle
- l'insert évaporateur (1) s'étend de l'ouverture d'insertion (42) à l'ouverture de sortie (74),
**caractérisée en ce que**
l'insert évaporateur (1), en particulier en forme de cartouche ou de manchon, peut être disposé dans une partie externe, à savoir dans le réservoir de liquide (30), et être poussé dans celui-ci, l'insert évaporateur (1) étant maintenu dans l'unité évaporateur-réservoir (26) de manière à ne pas pouvoir tourner, dans laquelle
- l'évaporateur (3) comprend un corps chauffant (60) en forme de bloc en un matériau électriquement conducteur, dans laquelle
- le corps chauffant (60) est pourvu d'une pluralité de micro-canaux (62) qui relient un côté entrée (61) du corps chauffant (60) à un côté sortie (64) de manière à permettre la circulation de liquide, dans laquelle
- le côté entrée (61) est relié au réservoir de liquide (30) de manière à permettre la circulation de liquide par l'intermédiaire d'une structure de mèche ou d'un élément d'amenée de liquide (16), dans laquelle
- l'élément d'amenée de liquide (16) sert à transporter passivement du liquide (2) d'un réservoir de liquide (30) vers le corps chauffant (60) au moyen de forces capillaires.

2. Unité évaporateur-réservoir (26) selon la revendication 1, **caractérisée en ce que**
- l'insert évaporateur (1) comprend au moins un élément de maintien et/ou de fixation (38) pour maintenir et/ou fixer l'insert évaporateur (1) dans la partie externe (30).

3. Unité évaporateur-réservoir (26) selon la revendication 1 ou 2, **caractérisée en ce que**
- l'insert évaporateur (1) peut être installé dans la partie externe (30) de manière à ne pas pouvoir tourner.

4. Unité évaporateur-réservoir (26) selon l'une des revendications précédentes, **caractérisée en ce que**
- les contacts électriques (10) sont disposés sur l'une des faces d'extrémité (11, 12) de l'insert évaporateur (1).

5. Unité évaporateur-réservoir (26) selon l'une des revendications précédentes, **caractérisée en ce que**
- au moins un dispositif d'amenée et/ou d'évacuation d'air perméable à l'air (17) est prévu sur la face d'enveloppe de la partie de base (14).

6. Unité évaporateur-réservoir (26) selon l'une des revendications précédentes, **caractérisée en ce que**
- le canal d'écoulement (13) comprend au moins une zone de pré- et/ou post-traitement (18) pour le pré- et/ou post-traitement de l'air et/ou du liquide (2) évaporé s'écoulant dans le canal d'écoulement (13).

7. Unité évaporateur-réservoir (26) selon l'une des revendications précédentes, **caractérisée en ce que**
- l'insert évaporateur (1) comprend une pluralité d'évaporateurs électriques (3a, 3b).

8. Unité évaporateur-réservoir (26) selon la revendication 7, **caractérisée en ce que**
- les évaporateurs électriques (3a, 3b) sont disposés le long de la circonférence de la face d'enveloppe (31) de la partie de base (14) et/ou le long du canal d'écoulement (13).

9. Unité évaporateur-réservoir (26) selon l'une des revendications 7 ou 8, **caractérisée en ce que**
- l'insert évaporateur (1) présente une pluralité d'ouvertures pour liquide (15a, 15b), dans laquelle
- chaque évaporateur électrique (3a, 3b) peut être alimenté en liquide (2a, 2b) à partir d'une ouverture pour liquide (15a, 15b) correspondante.

10. Unité évaporateur-réservoir (26) selon l'une des revendications précédentes, **caractérisée en ce que**
- l'insert évaporateur (1) présente sur la face d'enveloppe (31) de la partie de base (14) au moins un élément de maintien, en particulier un éperon (32), pour maintenir un élément capillaire (33) dans un réservoir de liquide (30).

11. Unité évaporateur-réservoir (26) selon l'une des revendications précédentes, **caractérisée en ce que**
- l'insert évaporateur (1) comprend au moins un canal d'air secondaire (80) séparé du canal d'écoulement (13).

12. Unité évaporateur-réservoir (26) selon l'une des revendications précédentes, **caractérisée en ce que**
- l'ouverture pour liquide (15) communique avec un élément d'amenée de liquide (16), dans laquelle
- l'élément d'amenée de liquide (16) est conçu pour transporter le liquide (2) vers l'évaporateur (3).

13. Unité évaporateur-réservoir (26) selon l'une des revendications précédentes, **caractérisée en ce que**
- l'insert évaporateur (1) comprend une unité électronique (4) pour la commande et/ou la régulation de l'évaporateur (3) et/ou pour le stockage de données caractéristiques.

14. Unité évaporateur-réservoir (26) selon l'une des revendications précédentes, **caractérisée en ce que** la partie de base (14) est en forme de cartouche ou de manchon.

15. Unité évaporateur-réservoir (26) selon l'une des revendications précédentes, **caractérisée en ce que**
- le réservoir de liquide (30) entoure la face d'enveloppe (31) de l'insert évaporateur (1) au moins dans la section de l'ouverture pour liquide (15).

16. Unité évaporateur-réservoir (26) selon l'une des revendications précédentes, **caractérisée en ce que**
- un élément capillaire (33) est associé à l'ouverture pour liquide (15) à l'intérieur du réservoir de liquide (30).

17. Unité évaporateur-réservoir (26) selon l'une des revendications précédentes, **caractérisée en ce que**
- le réservoir de liquide (30) comprend une pluralité de chambres (30a, 30b) séparées les unes des autres, et
- chaque évaporateur électrique (3a, 3b) peut être alimenté en liquide (2a, 2b) à partir d'une chambre (30a, 30b) correspondante.

18. Unité évaporateur-réservoir (26) selon l'une des revendications précédentes, **caractérisée en ce que**
- au moins une des faces d'extrémité (11, 12) de l'insert évaporateur (1) est alignée avec une face d'extrémité (65, 66) correspondante du réservoir de liquide (30).

19. Unité évaporateur-réservoir (26) selon l'une des revendications précédentes, **caractérisée en ce que**
- le réservoir de liquide (30) présente au moins un élément d'amenée et/ou d'évacuation d'air perméable à l'air (47).

20. Inhalateur (27) comprenant une unité évaporateur-réservoir (26) selon l'une des revendications précédentes, et un dispositif de stockage d'énergie (55) pour l'alimentation électrique dudit au moins un évaporateur (3).

21. Procédé de fabrication d'une unité évaporateur-réservoir (26) selon l'une des revendications 1 à 19 et/ou d'un inhalateur (27) selon la revendication 20, comprenant la mise à disposition de l'insert évaporateur (1) et du réservoir de liquide (30), **caractérisé en ce que** l'insert évaporateur (1) est inséré, à savoir poussé, dans l'ouverture d'insertion (42) et jusqu'à l'ouverture de sortie (74) du réservoir de liquide (30).

22. Procédé selon la revendication 21, **caractérisé en ce que**
- l'insert évaporateur (1) est guidé, à savoir poussé, avec la face d'extrémité (11) côté sortie jusqu'à l'ouverture de sortie (74) ;
- le réservoir de liquide (30) est rempli d'un liquide (2) par une ouverture de remplissage (73) disposée ou restant entre la partie de base (14) et l'ouverture d'insertion (42) ; et
- l'insert évaporateur (1) est guidé, à savoir poussé, à travers l'ouverture d'insertion (42) jusqu'à une butée, fermant ce faisant l'ouverture de remplissage (73).

23. Procédé selon l'une des revendications 21 ou 22, **caractérisé en ce que** l'insert évaporateur (1) est fabriqué sous la forme d'un module qui peut être amené ou est amené à un flux de fabrication d'une unité évaporateur-réservoir (26) et/ou d'un inhalateur (27).
